# EUROPEAN PATENT SPECIFICATION

(11) **EP 2 518 081 B1**
(45) Date of publication and mention of the grant of the patent: **29.11.2017**
(21) Application number: 11164083.5
(22) Date of filing: 28.04.2011
(51) Int. Cl.: C07K 14/435, C12N 15/82, D01F 4/00, C07K 1/30, C07K 1/34

(54) **Method of producing and purifying polymeric proteins in transgenic plants**
Verfahren zur Herstellung und Reinigung von Polymerproteinen in transgenen Pflanzen
Procédé pour la production et la purification de protéines polymères dans des plantes transgéniques

(43) Date of publication of application: 31.10.2012
(73) Proprietor: Leibniz-Institut für Pflanzengenetik und Kulturpflanzenforschung (IPK), 06466 Gatersleben (DE)
(72) Inventor: Hauptmann, Valeska, 06493 Straßberg (DE); Gils, Mario, 06484 Quedlinburg (DE); Conrad, Udo, 06458 Hausneindorf (DE); Phan, Hoang Trong, 06466 Gatersleben (DE)
(74) Representative: Lasar, Andrea Gisela

(56) References cited:
- WO-A2-01/94393
- WO-A2-2008/024311
- WO-A2-2009/147179
- US-A1- 2002 177 691
- Schallau, Kai: "Herstellung von Spinnenseidenproteinen in Tabaksamen", , 2008, XP002670108, Retrieved from the Internet: URL:http://sundoc.bibliothek.uni-halle.de/ diss-online/08/08H175/prom.pdf [retrieved on 2012-02-21]
- YANG J ET AL: "Intein-mediated assembly of a functional beta-glucuronidase in transgenic plants", PROCEEDINGS OF THE NATIONAL ACADEMY OF SCIENCES, NATIONAL ACADEMY OF SCIENCES, WASHINGTON, DC; US, vol. 100, no. 6, 18 March 2002 (2002-03-18), pages 3513-3518, XP002967417, ISSN: 0027-8424, DOI: 10.1073/PNAS.0635899100
- X.-X. XIA ET AL: "Native-sized recombinant spider silk protein produced in metabolically engineered Escherichia coli results in a strong fiber", PROCEEDINGS OF THE NATIONAL ACADEMY OF SCIENCES, vol. 107, no. 32, 10 August 2010 (2010-08-10), pages 14059-14063, XP55006380, ISSN: 0027-8424, DOI: 10.1073/pnas.1003366107
- DATABASE BIOSIS [Online] BIOSCIENCES INFORMATION SERVICE, PHILADELPHIA, PA, US; February 2004 (2004-02), SCHELLER JUERGEN ET AL: "Purification of spider silk-elastin from transgenic plants and application for human chondrocyte proliferation.", XP055015164, Database accession no. PREV200400265560 & TRANSGENIC RESEARCH, vol. 13, no. 1, February 2004 (2004-02), pages 51-57, ISSN: 0962-8819
- GE XIN ET AL: "Purification of an elastin-like fusion protein by microfiltration", BIOTECHNOLOGY AND BIOENGINEERING, WILEY & SONS, HOBOKEN, NJ, US, vol. 95, no. 3, 20 October 2006 (2006-10-20), pages 424-432, XP009126250, ISSN: 0006-3592
- Wafa Hassouneh ET AL: "Elastin-Like Polypeptides as a Purification Tag for Recombinant Proteins" In: "Current Protocols in Protein Science", 1 August 2010 (2010-08-01), John Wiley & Sons, Inc., Hoboken, NJ, USA, XP55020030, ISBN: 978-0-47-114086-3 * abstract; pages 6.11.1, 6.11.3, 6.11.5, 6.11.7, 6.11.11 and 6.11.13 *
- HOANG TRONG PHAN ET AL: "Membrane-Based Inverse Transition Cycling: An Improved Means for Purifying Plant-Derived Recombinant Protein-Elastin-Like Polypeptide Fusions", INTERNATIONAL JOURNAL OF MOLECULAR SCIENCES, vol. 12, no. 5, 29 April 2011 (2011-04-29) , pages 2808-2821, XP55019944, DOI: 10.3390/ijms12052808
- HAUPTMANN, V. ET AL.: "Native-sized spider silk proteins synthesized in palnta via intein-based multimerization", TRANSGENIC RESEARCH, vol. 22, no. 2, 22 September 2012 (2012-09-22), pages 369-377,
- OLSEN, D.: "Recombinant collagen and gelatin for drug delivery", ADVANCED DRUG DELIVERY REVIEWS, vol. 55, no. 12, 28 November 2003 (2003-11-28), pages 1547-1567, XP002368792,

## Description

### FIELD OF THE INVENTION

The present invention relates to a method of producing polymeric proteins in transgenic plants using intein-mediated trans-splicing, as well as to polymeric proteins obtained thereby, recombinant nucleic acid molecules and transgenic plants useful in this method. The present disclosure further relates to a method of purifying the polymeric protein using membrane-based inverse transition cycling.

### BACKGROUND OF THE INVENTION

Polymeric proteins comprising repeating units of specific amino acid sequences such as silk proteins, seed storage proteins and collagen have useful properties due to the repetitive amino acid sequences.

For example, the polymeric spider silk proteins (spidroins) MaSp1 and MaSp2 are the main components of the spider dragline silk which is exceptionally strong and elastic. These characteristics of the spider silk are conferred by the repetitive sequences within the spider silk proteins. Due to its unique mechanical properties, spider dragline silk is used in many industrial and biomedical applications, for example as sutures for wounds, adhesion material in cell culture, scaffolds for artificial organs, in parachute cords and protective clothing.

Another example of polymeric proteins are the glutenins which are synthesized and stored in the endosperm cells of the seeds. These proteins are also elastomeric due to the repetitive amino acid sequences. Hence, these proteins are already used in the polymer industry with applications in biomedicine and nanobiotechnology.

To use the above and other polymeric proteins in industrial processes it is necessary to produce and purify them in a large scale. Nowadays, proteins are often produced using recombinant DNA technology. However, proteins with repetitive amino acid sequences are difficult to express, as the repetitive DNA sequences from which they are expressed are often subject to genetic recombination resulting in genetic instability. Hence, often proteins with a size less than the native size of the protein are produced (see, e.g., WO 01/94393; Lazaris et al. (2002) Science 295: 472-476; Saumonneau et al. (2011) Plant Cell Rep., published online on 5 March 2011).

Nevertheless, it is desirable to produce native-sized polymeric proteins, as for example for spider silk proteins it has been shown that the size of the protein is important for controlling the mechanical properties of the spun fiber (Xia et al. (2010) Proc. Natl. Acad. Sci. USA 107(32): 14059-14063).

One approach to produce native-sized spider silk protein was to use a metabolically engineered *E. coli* host in which the glycyl-tRNA pool was elevated, as the spider silk proteins produced in this study are extremely rich in glycine (Xia et al. (2010) Proc. Natl. Acad. Sci. USA 107(32): 14059-14063). However, this approach is quite laborious and may not be applicable to other polymeric proteins which are less rich in glycine than the spider silk proteins tested in this study.

Another possibility would be to isolate the protein monomers from the host cells and to fuse them *in vitro.* However, this approach requires the purification of the protein monomers to be fused and the efficient removal of the agents supporting the fusion.

US 2002/0177691 A1 suggests to use intein-mediated splicing for assembling monomers to polymeric proteins. However, it does not disclose that this method can be performed in plant cells. Further, it does not disclose a method with which the polymer can be efficiently recovered from the host cells.

WO 2009/147179 A2 discloses a method for producing male sterile monocotyledonous plants by intein-mediated splicing of a nucleic acid sequence encoding a protein which mediates male sterility.

Hence, there is still a need for an efficient method for producing and purifying polymeric proteins.

### OBJECT AND SUMMARY OF THE INVENTION

It is thus an object of the present invention to provide a method for producing and, eventually, purifying polymeric proteins with a high efficiency and a high purity.

These and further objects of the invention, as will become apparent from the description, are attained by the subject-matter of the independent claims.

Some of the preferred embodiments of the present invention form the subject-matter of the dependent claims.

The present inventors have found that polymeric proteins can be efficiently produced in transgenic plants by using intein-mediated trans-splicing to assemble the monomers into the polymeric protein. Further, the production of fusion proteins of the protein monomers and ELP enables the purification of the protein polymers with high yield and a high purity using a new purification method for ELP fusion proteins.

Accordingly, in one embodiment, the present invention provides a method of producing a recombinant polymeric protein in transgenic plants or plant cells, comprising the steps of:
a) introducing into the plant or plant cell at least one recombinant nucleic acid molecule comprising the following elements in 5' to 3' orientation:
   - a promoter functional in plant cells;
   - operatively linked thereto a nucleic acid sequence coding for the C-terminal part of an intein;
   - a nucleic acid sequence coding for a monomer of the polymeric protein;
   - a nucleic acid sequence coding for the N-terminal part of an intein; and
   - optionally, operatively linked thereto a terminator sequence functional in plant cells;
   wherein the recombinant nucleic acid molecule further comprises a nucleic acid sequence coding for 10 to 100 repeats of the pentapeptide Val-Pro-Gly-Xaa-Gly (ELP), wherein Xaa can be any amino acid except proline, which nucleic acid sequence is located 5' or 3' of or within the nucleic acid sequence coding for the monomer of the polymeric protein, wherein the nucleic acid sequence coding for the ELP is positioned next to the nucleic acid sequence coding for the monomer of the polymeric protein so that a single open reading frame is created;
b) optionally, regenerating fertile plants from the transformed cells; and
c) isolating the recombinant polymeric protein from the transgenic plant or plant cells.

In one embodiment, one recombinant nucleic acid molecule as described above is introduced into the plant or plant cell and the C-terminal part of the intein and the N-terminal part of the intein are derived from the same intein.

In an alternative embodiment, two recombinant nucleic acid molecules as described above are introduced into the plant or plant cell, wherein the first recombinant nucleic acid molecule comprises the C-terminal part of a first intein and the N-terminal part of a second intein and wherein the second recombinant nucleic acid molecule comprises the C-terminal part of said second intein and the N-terminal part of said first intein.

In a preferred embodiment of the present invention the recombinant polymeric protein is selected from the group consisting of spider silk proteins, fibroins, seed storage proteins and collagen.

In a further preferred embodiment of the present invention the step of isolating the recombinant polymeric protein comprises two filtration steps, wherein the first filtration step is performed in the presence of NaCl and at a temperature between 2°C and a temperature which is at least 5°C below the inverse transition temperature of the fusion protein. More preferably, the protein is eluted at a temperature of 2°C to 8°C after the second filtration step.

The invention further relates to a recombinant polymeric protein obtainable by the method of the present invention, wherein the recombinant polymeric protein has a molecular weight of at least 180kD and a structure selected from the group of
a) (monomer-ELP)ₓ,
b) (ELP-monomer)ₓ or
c) monomerₓ, wherein ELP is located within the monomer,
wherein x is 2 to 25 and wherein ELP means 10 to 100 repeats of the pentapeptide Val-Pro-Gly-Xaa-Gly (ELP), wherein Xaa can be any amino acid except proline.

In a preferred embodiment, the recombinant polymeric protein is a spider silk protein.

In a further embodiment the present invention relates to the use of the spider silk protein produced by the method of the present invention for producing synthetic threads, films and/or membranes.

In a further aspect, the invention provides a recombinant nucleic acid molecule, comprising the following elements in 5' to 3' orientation:
- a promoter functional in plant cells;
- operatively linked thereto a nucleic acid sequence coding for the C-terminal part of an intein;
- a nucleic acid sequence coding for a monomer of a polymeric protein;
- a nucleic acid sequence coding for the N-terminal part of an intein; and
- optionally, operatively linked thereto a terminator sequence functional in plant cells;
wherein the recombinant nucleic acid molecule further comprises a nucleic acid sequence coding for 10 to 100 repeats of the pentapeptide Val-Pro-Gly-Xaa-Gly (ELP), wherein Xaa can be any amino acid except proline, which nucleic acid sequence is located 5' or 3' of or within the nucleic acid sequence coding for the monomer of the polymeric protein.

In still a further aspect, the present invention provides a transgenic plant comprising a recombinant nucleic acid molecule of the present invention as well as transgenic parts of this plant comprising a recombinant nucleic acid molecule of the present invention, transgenic harvest products of this plant comprising a recombinant nucleic acid molecule of the present invention and transgenic propagating material of this plant comprising a recombinant nucleic acid molecule of the present invention, such as protoplasts, plant cells, calli, seeds, tubers, cuttings, and the transgenic progeny of this plant comprising a recombinant nucleic acid molecule of the present invention.

Further, the present disclosure relates to a method of isolating an ELP fusion protein from transgenic plants, comprising two filtration steps, wherein the first filtration step is performed in the presence of NaCl and at a temperature between 2°C and a temperature which is at least 5°C below the inverse transition temperature of the fusion protein. More preferably the protein is eluted from the filter membrane at a temperature of 2°C to 8°C after the second filtration step.

### BRIEF DESCRIPTION OF THE DRAWINGS

Figure 1: Vector constructs useful in the present invention
   a) pCB301pHW401 Flag for the production of FLAG polymers
   b) pCB301pHW401 FlagELP for the production of FLAG-ELP intein fusions
   c) pCB301pHW401 GlutELP for the production of Glutenin-ELP intein fusions
Figure 2: Western blot analysis of FLAG multimers expressed in transgenic tobacco leaves
   20 µg extract from leaves (0.28g leaves/ml buffer) of transgenic tobacco plants transformed with the vector pCB301pHW401 Flag-Intein was separated on a 3-12% SDS-PAA gradient gel and immunoblotted against anti-c-myc antibody. The arrows indicate the positions of the multimers comprising different numbers of monomers.

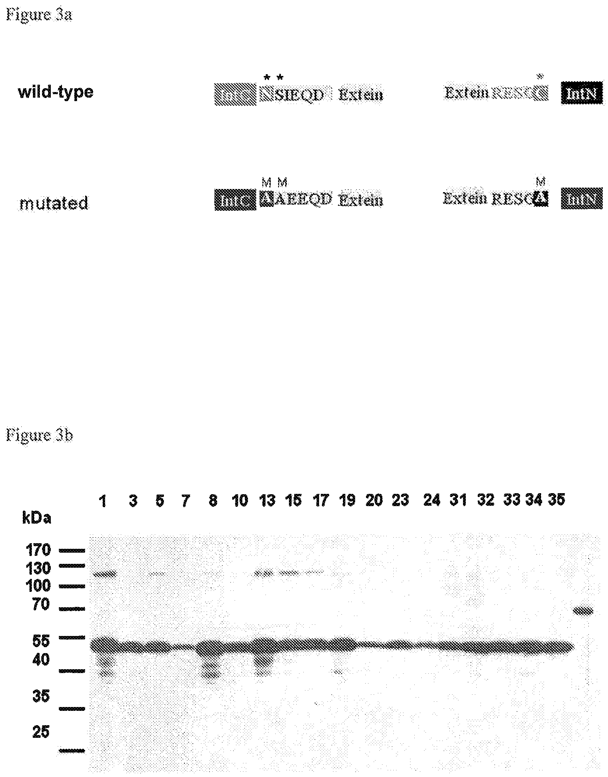

Figure 3: Protein expression from a vector comprising mutated extein sequences.
   a) Schematic drawing of the mutated extein sequences.
      Intein regions are shown with black background and extein regions are shown with grey background. Conserved amino acids in the wild-type exteins are labeled with an asterisk, while the mutated amino acids in the mutated exteins are labeled with "M".
   b) Western blot analysis of the FLAG protein expressed from a vector comprising mutated exteins (pCB301pHW401 FLAG-Inteinmut) in transgenic tobacco plants. 10 µg extract from different transgenic TG0 tobacco lines transformed with pCB301pHW401 FLAG-Inteinmut were separated on a 12% SDS-polyacrylamide gel and immunoblotted against anti-c-myc antibody.

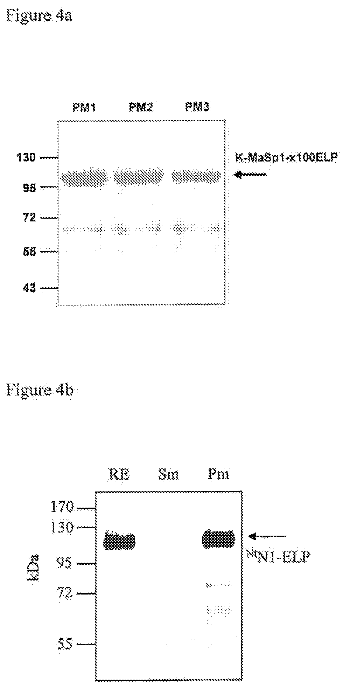

Figure 4: Analysis of ELP fusion proteins purified by the method of the present invention.
   a) Gel analysis of MaSP1-ELP fusion proteins purified by the method of the present invention.
      An extract from transgenic tobacco plants expressing MaSp1-ELP (Scheller et al. (2004) Transgenic Res. 13: 51-57) was purified as described in Example 3 and separated on a 10% SDS-polyacrylamide gel which was then stained with Coomassie blue. The position of the MaSp1-ELP fusion protein is indicated with an arrow.
   b) Gel analysis of N1-ELP fusion proteins purified by the method of the present invention
      An extract from transgenic tobacco plants expressing N1-ELP was purified as described in Example 4 and separated on a 10% SDS-polyacrylamide gel and immunoblotted against anti-c-myc antibody. RE: raw extract; Sm: supernatant raw extract passed through a 0.2µm cellulose acetate membrane at room temperature; Pm: proteins eluted from the membrane by ice-cold water. The arrow indicates the target recombinant protein N1.

### DETAILED DESCRIPTION OF THE INVENTION

The present invention as illustratively described in the following may suitably be practiced in the absence of any element or elements, limitation or limitations, not specifically disclosed herein.

The present invention will be described with respect to particular embodiments but the invention is not limited thereto but only by the claims.

Where the term "comprising" is used in the present description and claims, it does not exclude other elements. For the purposes of the present invention, the term "consisting of' is considered to be a preferred embodiment of the term "comprising". If hereinafter a group is defined to comprise at least a certain number of embodiments, this is also to be understood to disclose a group which preferably consists only of these embodiments.

For the purposes of the present invention, the term "obtained" is considered to be a preferred embodiment of the term "obtainable". If hereinafter e.g. a protein is defined to be obtainable by a specific method, this is also to be understood to disclose a protein which is obtained by this method.

Where an indefinite or definite article is used when referring to a singular noun, e.g. "a", "an" or "the", this includes a plural of that noun unless something else is specifically stated.

The term "polymeric protein" refers to a protein which comprises at least two identical monomers with a defined amino acid sequence which monomers are covalently linked to form the polymer. The polymeric protein produced by the method of the present invention comprises 2 to 25, preferably 2 to 20, more preferably 2 to 15 and most preferably 2 to 12 repeats of the monomeric amino acid sequence. The polymeric protein produced by the method of the present invention has a molecular weight of at least 70 or 80 kD, preferably at least 100 or 120 kD, more preferably at least 150 or 180 kD and most preferably at least 200 to 250 kD. The skilled person will appreciate that the polymeric protein produced by the method of the present invention is present as a mixture of multimers having different numbers of repeats of the monomer and different molecular weight. For example, the polymeric protein produced by the method of the present invention can be a mixture of polymeric proteins, each polymeric protein comprising a different number of monomers such as two, three, four, five, six, seven, eight, nine, ten or more monomers.

The "monomer" of the polymeric protein comprises the amino acid sequence which is repeated in the polymeric protein produced by the method of the present invention. Within the scope of the present invention, the monomer may not only comprise those sequences which are repeated within the naturally occurring polymeric protein, but may also comprise additional sequences from the naturally occurring polymeric protein which are not repeated within the naturally occurring polymeric protein, but which are repeated in the polymeric protein produced by the method of the present invention.

Polymeric proteins which can be produced by the method of the present invention include, but are not limited to, spider silk proteins, fibroins, seed storage proteins, abductin, resilin and collagen.

In a preferred embodiment of the present invention the polymeric proteins produced by the method of the present invention are selected from spider silk proteins, fibroins and seed storage proteins.

"Spider silk proteins" are components of the protein fibre spun by spiders. The dragline silk is primarily composed of two proteins, the major ampullate spidroins 1 (MaSp1) and 2 (MaSp2). Further, one component of the capture-spiral silk is the Flag protein. Within the scope of the present invention, the term "spider silk protein" comprises both the naturally occurring proteins of the spider silk, e.g. MaSp1 MaSp2, and synthetic proteins which have a high similarity to the naturally occurring spider silk proteins and which exhibit the same characteristics as the naturally occurring protein. Synthetic spider silk proteins are described for example in WO 01/94393 A2.

Preferably, the spider silk protein has a monomer sequence according to SEQ ID No. 1 (so-called FLAG monomer) or according to SEQ ID No. 2 (so-called SO1 monomer). Also preferably, the spider silk protein is MaSp1 having the monomer sequence according to SEQ ID No. 3 or MaSp2 having the monomer sequence according to SEQ ID No. 4. The skilled person knows that it may be necessary to add an initiator methionine to the above amino acid sequences, if such initiator methionine is not present and the protein is expressed without a signal peptide located upstream of the monomer sequence.

"Fibroins" are proteins synthesized by insects such as the silkworm *Bombyx mori* which proteins are constituents of silk. An overview of monomer sequences of fibroins is provided in Craig and Riekel (2002) Comparative Biochemistry and Physiology Part B 133: 493-507 which is incorporated herein by reference.

"Seed storage proteins" are present in the seed of plants and serve as a source of food for the embryo during germination and early growth. Within the present invention, the term "seed storage protein" comprises polymeric proteins such as glutenins, triticins, HMW (high molecular weight) albumins and zeins.

Preferably, the seed storage protein is a glutenin and more preferably the monomer of the glutenin has the amino acid sequence according to SEQ ID No. 5.

Within the method of the present invention the polymeric protein or another protein which is to be purified is expressed as a fusion with ELP (elastin-like polypeptide). ELPs are oligomeric repeats of the pentapeptide Val-Pro-Gly-Xaa-Gly (wherein Xaa is every amino acid except proline and is preferably Gly), and are subjected to a reversible inverse temperature transition. They are very soluble in water below the inverse transition temperature (Tt), but have a sharp phase transition state in the range of 2°C to 3°C, when the temperature is increased to above Tₜ, which leads to precipitation and aggregation of the polypeptide. D.E. Meyer and A. Chilkoti ((1999) Nat. Biotech. 17: 1112-1115) have described that ELP fusions with recombinant proteins alter the solubility behaviour of these recombinant proteins at various temperatures and concentrations in a targeted fashion. It has also been shown that the fusion of a recombinant protein of interest with ELP enhances the yield of this protein produced in transgenic plants (Scheller et al. (2001) Nature Biotechnol. 19: 573-577; Patel et al. (2007) Transgenic Res. 16: 239-249). The use of ELP fusion proteins in recombinant protein expression is reviewed in Floss et al. (2010) Trends Biotechnol. 28(1): 37-45.

In the present invention, the fusion with ELP is used to establish purification strategies described in detail below for the polymeric proteins and other proteins of interest which are to be purified. The recombinant nucleic acid molecule of the present invention comprises a nucleic acid sequence coding for 10 to 100 repeats, preferably 50 to 100 and more preferably 70 to 100 repeats of the pentameric ELP units described above. The fusion of the polymeric protein with ELP is accomplished by positioning the nucleic acid sequence coding for the ELP next to the nucleic acid sequence coding for the monomer of the polymeric protein so that a single open reading frame is created.

Preferably, the nucleic acid sequence coding for 10 to 100 repeats of the pentameric ELP units is located 3' of the nucleic acid sequence coding for a monomer of the polymeric protein. More preferably, the nucleic acid sequence coding for 10 to 100 repeats of the pentameric ELP units is located 3' of the nucleic acid sequence coding for a monomer of the polymeric protein and the recombinant nucleic acid molecule further comprises at least one nucleic acid sequence that codes for a plant signal peptide directing the fusion protein to the endoplasmatic reticulum and at least one nucleic acid sequence coding for a retention sequence for the endoplasmatic reticulum. An example for locating the ELP within the monomer is disclosed in Saumonneau et al. (2011) Plant Cell Rep., published online on 5 March 2011.

The term "transgenic plant or plant cell" refers to a plant or plant cell which contains a foreign nucleic acid molecule which is not present in the naturally occurring, non-transgenic plant. Within the scope of the present invention, the foreign nucleic acid molecule is introduced into the plant or plant cell e.g. by transformation and is expressed within the transgenic plant or plant cell so that the protein it encodes is produced. In addition to the recombinant nucleic acid molecule encoding the monomer of the polymeric protein the transgenic plant or plant cell may contain further recombinant nucleic acid molecules, for example a recombinant nucleic acid molecule which encodes a protein conferring resistance to herbicides or pests.

Within the scope of the present invention, the term "recombinant nucleic acid molecule" means a nucleic acid molecule which contains all elements which are necessary for the expression of a gene or a fragment thereof, i.e. the gene to be expressed under the control of a suitable promoter and optionally further regulatory sequences such as termination sequences. The combination of elements within the recombinant nucleic acid molecule does not occur in nature. A recombinant nucleic acid molecule of the present invention may be part of an expression vector which is transferred into a plant cell or may be integrated into the chromosome of a transgenic plant after transformation. The terms "first recombinant nucleic acid molecule" and "second recombinant nucleic acid molecule " are only used to distinguish the different recombinant nucleic acid molecules comprising different elements, but are not intended to indicate any order between the recombinant nucleic acid molecules, i.e. the second recombinant nucleic acid molecule may be introduced into the plant or plant cell before the first recombinant nucleic acid molecule or the two nucleic acid molecules may be introduced simultaneously.

If the recombinant nucleic acid molecule is part of an expression vector, the expression vector is preferably selected from the group consisting of plasmids, cosmids, (recombinant) viruses and other vectors known in the field of gene technology, with which nucleic acid molecules can be transferred to plants or plant cells. The term "vector" also comprises so-called minichromosomes which are linear or circular DNA fragments which contain centromer sequences of the respective plant in addition to the transgene. Minichromosomes are stable in the nucleus and are passed on to the daughter cells during cell division. They are transferred by standard methods of transformation. Most preferably, the vector is selected from the group consisting of pBR322, pUC vectors, M13mp vectors or vectors being derived from the Ti plasmid or the Ri plasmid of agrobacteria.

Suitable cloning vectors contain a replication signal for *E. coli* and a marker gene for the selection of transformed bacterial cells. The required sequence can be introduced into the vector at an appropriate restriction site. The plasmid obtained is used for the transformation of *E. coli* cells. Transformed *E. coli* cells are cultivated in an appropriate medium, and finally harvested and lysed. The plasmid is recovered. To analyze the plasmid DNA obtained, methods such as restriction analyses, gel electrophoreses and other biochemical/molecular biological methods are available. Following each manipulation the plasmid DNA can be cleaved and the DNA fragments obtained can be combined with other DNA sequences. Each plasmid DNA sequence can be cloned into the same or other plasmids. Standard cloning methods can be taken from Sambrook et al., 2001 (Molecular cloning: A laboratory manual, 3rd edition, Cold Spring Harbor Laboratory Press).

The monomer of the polymeric protein is expressed under the control of a promoter which is functional in plant cells. Preferably, constitutive promoters such as the 35S promoter, the actin promoter, preferably the rice actin1 promoter (McElroy et al. (1990) Plant Cell 2: 163 - 171), or the ubiquitin promoter, preferably the maize ubiquitin promoter (Christensen et al. (1996) Transgenic Res. 5: 213-218) are used. However, other promoters can of course be used which are obtainable from different sources such as plants, plant viruses or fungi. The choice of promoter and other regulatory sequences determines the local and temporal expression pattern of the gene. Besides constitutive promoters, also tissue-specific promoters such as the phosphoenolpyruvate promoter or the fructose-1,6-bisphosphatase promoter or inducible promoters are conceivable, with seed-specific promoters such as the USP promoter (Zakharov et al. (2004) J. Exp. Bot. 55(402): 1463-1471) being preferred. More preferably, the CaMV 35S promoter is used to express the monomer.

The term "operatively linked" is understood to denote that the sequences linking the different nucleic acids used are selected in such a way that the function of the respectively linked nucleic acid segment is maintained. In case, for example, the nucleotide sequence coding for the monomer of the polymeric protein is to be expressed in a cell, it has to be ensured that no sequences which would lead to a termination of the transcription are located between the promoter sequence and the nucleotide sequence coding for the monomer of the polymeric protein. Further, it has to be ensured that the fusion protein is expressed by linking the protein components of the fusion protein with a peptide bond.

The "termination sequences" are sequences which ensure that the transcription or the translation is properly terminated. If the transferred nucleic acids are to be translated, the termination sequences are typically stop codons and corresponding regulatory sequences; if the transferred nucleic acids are only to be transcribed, they are generally poly-A sequences. Preferably, the termination sequence is selected from the CaMV 35S terminator, the octopine synthase terminator and the nopaline synthase terminator (Jones et al. (1992) Transgenic Res. 1: 285 - 297). If a CaMV 35S promoter is used to express the monomer of the polymeric protein, a CaMV 35S terminator is used to terminate transcription.

In a preferred embodiment, the recombinant nucleic acid molecule further comprises at least one nucleic acid sequence that codes for a plant signal peptide to ensure that the monomer is expressed and accumulated in suitable compartments of transgenic plants.

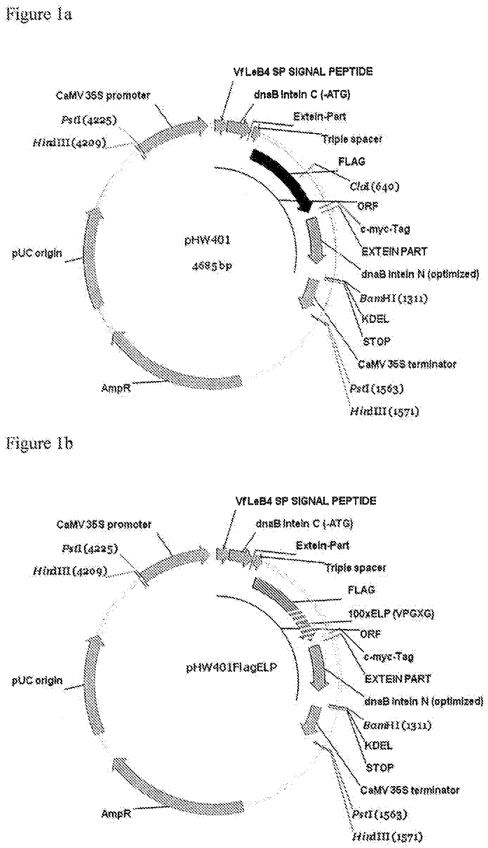

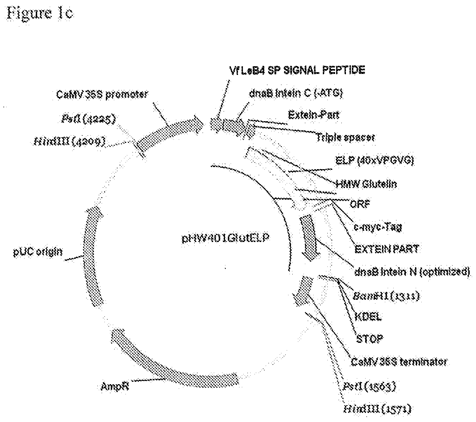

In a preferred embodiment, the endoplasmatic reticulum (ER) is the selected compartment for the expression or accumulation of the monomer. This compartment is particularly suitable for stable accumulation of foreign proteins in plants. To ensure transport into the ER, the nucleic acid molecule according to the invention preferably comprises corresponding signal peptides, the LeB4Sp sequence according to SEQ ID No. 13 being particularly preferred. The signal peptide is preferably localized at the N-terminus of the protein which is obtained by translating the nucleic acid sequences present in the nucleic acid molecule, i.e. N-terminal of the C-terminal part of the intein (see Figure 1).

ER retention, if desired, is ensured according to the invention in that the nucleic acid molecule according to the invention additionally comprises a nucleic acid sequence coding for an ER retention peptide. Retention in the ER is preferably achieved by the amino acid sequence KDEL (SEQ ID NO. 27) attached to the C terminus of the protein which is obtained by translating the nucleic acid sequences present in the nucleic acid molecule, i.e. C-terminal of the N-terminal part of the intein (see Figure 1).

Within the method of the present invention the peptide bond formation between the monomers to form the polymeric protein is accomplished by intein-mediated trans-splicing. For this purpose, the recombinant nucleic acid molecule further codes for inteins which are capable of mediating protein trans-splicing. "Inteins" are proteins that are able to catalyze trans-splicing events between separate protein monomers as they are able to excise themselves from precursor molecules and ligate the flanking protein sequences in a process termed protein-splicing (Southworth et al. (1998) EMBO J. 17: 918 - 926; Wu et al. (1998) Proc. Natl. Acad. Sci. USA 95: 9226 - 9231; Saleh and Perler (2006) Chem. Rec. 6: 183 - 193; Perler (1998) Cell 92: 1 - 4). By said trans-splicing, the monomers may be linked by peptide bond formation. Trans-splicing inteins may be selected from the nucleolar and organellar genomes of different organisms including eukaryotes, archaebacteria and eubacteria. Inteins that may be used for performing this invention are listed at http://www.neb.com/neb/inteins.html. The nucleotide sequence coding for an intein may be split into a 5' and a 3' part that code for the N-terminal and C-terminal part of the intein, respectively. Sequence portions not necessary for intein splicing (e.g. homing endonuclease domain) may be deleted. The intein coding sequence is split such that the 5' and the 3' parts are capable of trans-splicing. For selecting a suitable splitting site of the intein coding sequence, the considerations published by Southworth et al. (1998) EMBO J. 17: 918 - 926 may be followed. In constructing the recombinant nucleic acid molecule of the present invention, the 5' intein coding sequence is linked to the 3' end of the nucleic acid molecule coding for the monomer and the 3' intein coding sequence is linked to the 5' end of the nucleic acid molecule coding for the monomer.

Herein, peptide bond means the amide linkage between the carboxyl group of one polypeptide and the amino group of another polypeptide.

Preferably, the intein is selected from the group consisting of DnaB and DnaE, both from the green-blue algae of Synechocystis spec. More preferably, the intein is DnaB and most preferably, the N-terminal part of DnaB is encoded by the nucleic acid sequence according to SEQ ID No. 26 and the C-terminal part of DnaB is encoded by the nucleic acid sequence according to SEQ ID No. 16. Preferably, the N-terminal part of DnaB has the amino acid sequence according to SEQ ID No. 25 and the C-terminal part of DnaB has the amino acid sequence according to SEQ ID No. 15.

If two recombinant nucleic acid molecules are introduced into the plant or plant cell, the first intein may be DnaB and the second intein may be DnaE or the first intein may be DnaE and the second intein may be DnaB.

The recombinant nucleic acid molecule may further comprise a flexible linker sequence. The "flexible linker sequence" within the scope of the present invention is a short flexible peptide which is used to bridge the monomers of the polymeric protein without serious steric interference. The flexible linker sequence brings the two splice junctions in close proximity and helps to precisely align all reacting groups. Hence, efficient splicing is supported (Chong and Xu (1997) J. Biol. Chem. 272: 15587 - 15590). The most widely used linker designs have sequences consisting essentially of stretches of glycine (G) and serine (S) residues, because hydrophilic amino acids allow hydrogen bonding to the solvent and glycines provide the necessary flexibility. These properties prevent the penetration of the linker peptide into the hydrophobic interface formed in the association of the domains. Furthermore, the linkers are not able to form an ordered secondary structure. The term "essentially consist of glycine and serine residues" is intended to mean that at least 60% or 65%, preferably 70% or 75%, more preferably 80%, 82%, 84%, 86% or 88%, even more preferably 90%, 92%, 93%, 94%, 95%, 96%, 97%, 98% or 99% of the amino acid residues within the flexible linker sequence are glycine and/or serine residues. Most preferably, all amino acid residues within the flexible linker sequence are glycine and/or serine residues.

Most preferably, the flexible linker sequence has the amino acid sequence GGGGS (SEQ ID No. 19).

Preferably, stretches containing exon sequences are inserted between the C-terminal part of the intein and the N-terminal amino acid of the monomer of the polymeric protein and between the C-terminal amino acid of the monomer of the polymeric protein and the N-terminal part of the intein. As protein-splicing is dependent on the chemical nature of the splice-site junction amino acids, the insertion of stretches containing exon sequences is supposed to increase the efficiency of protein trans-splicing (Sun et al. (2001) Appl. Environ. Microbiol. 67: 1025 - 1029). Thus, the inserted exon sequences provide the parts of the exteins which are advantageous for the function of the corresponding intein.

Hence, preferably a nucleic acid sequence coding for an amino acid sequence comprising the amino acid sequence SIEQD or fragments of said sequences from the DnaB extein is inserted between the nucleic acid sequence coding for the C-terminal part of the intein and the nucleic acid sequence coding for the monomer of the polymeric protein. Preferably, a nucleic acid sequence coding for an amino acid sequence comprising the amino acid sequence SIEQD or fragments of said sequence is inserted between the nucleic acid sequence coding for the C-terminal part of the intein and the nucleic acid sequence coding for the monomer of the polymeric protein. "Fragments of the SIEQD extein sequence" may be for example IEQD, EQD or QD.

Also preferably, a nucleic acid sequence coding for an amino acid sequence comprising the amino acid sequence RESG or fragments of said sequence from the DnaB extein is inserted between the nucleic acid sequence coding for the monomer of the polymeric protein and the nucleic acid sequence coding for the C-terminal part of the intein. Fragments of the RESG extein sequence" may be for example ESG or SG. "An amino acid sequence comprising the amino acid sequence RESG" or " an amino acid sequence comprising the amino acid sequence SIEQD" may be an amino acid sequence with one or more amino acid residues in addition to the RESG and SIEQD sequence, respectively. Preferably, the one or more additional amino acid residues are located in the N-terminus of the RESG and SIEQD sequence, respectively.

In a most preferred embodiment the recombinant nucleic acid molecule comprises the following elements in 5' to 3' orientation:
- a promoter functional in plant cells;
- operatively linked thereto a nucleic acid sequence coding for a signal peptide which mediates transport into the endoplasmatic reticulum;
- a nucleic acid sequence coding for the C-terminal part of an intein;
- a nucleic acid sequence coding for the extein sequence SIEQD of the intein;
- a nucleic acid sequence coding for three copies of a flexible linker sequence;
- a nucleic acid sequence coding for a monomer of the polymeric protein;
- a nucleic acid sequence coding for 10 to 100 repeats of the pentapeptide Val-Pro-Gly-Xaa-Gly (ELP), wherein Xaa can be any amino acid except proline;
- optionally, a nucleic acid sequence coding for a protein tag;
- a nucleic acid sequence coding for the extein sequence RESG of the intein;
- a nucleic acid sequence coding for the N-terminal part of the intein;
- a nucleic acid sequence coding for an ER retention peptide; and
- optionally, operatively linked thereto a terminator sequence functional in plant cells.

According to one embodiment of the present invention, two recombinant nucleic acid molecules as described before are introduced into the plant or plant cell, wherein the first recombinant nucleic acid molecule comprises the C-terminal part of a first intein such as DnaB and the N-terminal part of a second intein such as DnaE and wherein the second recombinant nucleic acid molecule comprises the C-terminal part of said second intein and the N-terminal part of said first intein. It is noted that the recombinant nucleic acid molecules can also differ, e.g. with respect to the promoter or the terminator sequence. However, in a preferred embodiment the recombinant nucleic acid molecules only differ with respect to the inteins and the other components are identical.

For the introduction of DNA into a plant host cell there are a number of well-known techniques available and the person skilled in the art can determine the appropriate method in each case without any problem. These techniques include the transformation of plant cells with T-DNA by using *Agrobacterium tumefaciens* or *Agrobacterium rhizogenes* as a transformation agent, the fusion of protoplasts, the direct gene transfer of isolated DNA into protoplasts, the electroporation of DNA, the introduction of DNA by means of the biolistic method, as well as other possibilities. Thereby, both stable and transient transformants can be generated.

For the injection and electroporation of DNA into plant cells there are no special requirements per se for the plasmids used. The same applies for direct gene transfer. Simple plasmids, such as pUC derivates may be used. If, however, whole plants are to be regenerated from such transformed cells, the presence of a selectable marker gene is necessary. The person skilled in the art is acquainted with the current selection markers, and will have no problem in selecting an appropriate marker. Standard selection markers are those which mediate resistance to a biocide or an antibiotic such as kanamycin, G418, bleomycin, hygromycin, methotrexate, glyphosate, streptomycin, sulfonyl urea, gentamycin or phosphinotricin and suchlike, to the transformed plant cell.

Dependent upon the method of introduction of the desired gene into the plant cell, other DNA sequences may be required. If, for example, the Ti or Ri plasmid is used for the transformation of the plant cell, at least the right flanking region, often however the right and the left flanking region of the T-DNA contained in the Ti or Ri plasmid must be linked as a flanking region with the gene to be introduced.

If agrobacteria are used for the transformations, the DNA to be introduced must be cloned in special plasmids, either in an intermediary vector which cannot replicate in agrobacteria or in a binary vector which can replicate both in *E. coli* and in agrobacteria. The agrobacterium serving as a host cell should contain a plasmid which carries a vir region. The vir region is necessary for the transfer of the T-DNA into the plant cell. T-DNA can also be present. This type of transformed agrobacterium is used for the transformation of plant cells. The use of T-DNA for the transformation of plant cells has been intensively investigated and is sufficiently described in EP 0 120 515 A2. Both monocotyledonous and dicotyledonous plants or their cells are very accessible to transformation by means of vectors based on agrobacteria (Chan et al. (1993) Plant Mol. Biol. 22: 491-506).

For the transfer of DNA into the plant cell, plant explants can be cultivated specifically for this purpose with *Agrobacterium tumefaciens* or *Agrobacterium rhizogenes.* From the infected plant material (for example, pieces of leaf, stem segments, roots, but also protoplasts or suspension-cultivated plant cells) whole plants can be regenerated in an appropriate medium which can contain antibiotics or biocides for the selection of transformed cells. The regeneration of the plants takes place according to standard regeneration methods and using the common nutrient solutions. The plants and plant cells obtained in this way can be examined for the presence of the DNA introduced.

The person skilled in the art is acquainted with other possibilities for the introduction of foreign DNA, in particular into monocotyledonous plants or their cells, using the biolistic method (Wan and Lemaux (1994) Plant Physiol. 104: 37-48; Vasil et al. (1993) Bio/Technology 11: 1553-1558; Ritala et al. (1994) Plant Mol. Bio. 24: 317-325; Spencer et al. (1990) Theor. Appl. Genet. 79: 625-631), by protoplast transformation (see L. Willmitzer (1993) Transgenic Plants in: Biotechnology, A Multi-Volume Comprehensive Treatise (publisher: H.J. Rehm et al.), volume 2, 627-659, VCH Weinheim, Germany), electroporation of partially permeabilised cells as well as the introduction of DNA by means of glass tissues.

The transformed cells grow within the plant in the normal way (see also McCormick et al. (1986) Plant Cell Reports 5: 81-84). The resulting plants can be raised in the normal way and be crossed with plants which have the same transformed genetic disposition or other genetic dispositions. The resulting hybrid individuals have the respective phenotypical properties.

Two or more generations should be raised in order to ensure that the phenotypical feature remains stable and is inherited. Seeds should be harvested as well so as to ensure that the respective phenotype or other characteristics are maintained.

After introducing the expression cassettes into plant cells, transgenic plants can be identified by conventional methods such as PCR or Southern Blot analysis of plant material. Further, expression of the polymeric protein in the transgenic plants or plant cells can be monitored for example by Western Blot analysis.

The method of the present invention is suitable for producing polymeric proteins both in monocotyledonous and dicotyledonous plants. Preferably, the plant is a monocotyledonous or dicotyledonous crop plant such as a food or fodder plant or any other plants. Examples for monocotyledonous plants are plants belonging to the genera Avena (oat), Triticum (wheat), Secale (rye), Hordeum (barley), Oryza (rice), Panicum, Pennisetum, Setaria, Sorghum (millet), Zea (maize), Lolium and the like.

Examples of dicotyledonous plants include, *inter alia,* cotton, legumes, soy bean, rapeseed, tomato, sugar beet, potato, ornamental plants, and trees. Further useful plants can comprise fruit (in particular apples, pears, cherries, grapes, citrus, pineapple, and bananas), pumpkin, cucumber, wine, oil palms, tea shrubs, cacao trees, and coffee shrubs, tobacco, sisal, as well as, with medicinal plants, rauwolfia and digitalis.

In the method of the present invention preferably tobacco, barley or alfalfa plants are used.

The polymeric proteins produced by intein-mediated trans-splicing and other ELP fusion proteins may be purified by a novel purification method developed by the present inventors. This method involves a first and a second filtration step wherein the first filtration step is performed in the presence of NaCl at a temperature between 2°C and a temperature which is at least 5°C below the inverse transition temperature, preferably between 2°C and 8°C and most preferably at 4°C. By using these conditions in the first filtration step the ELP fusion protein remains in solution, whereas contaminating proteins are precipitated. Consequently, an ELP fusion protein with a high purity is obtained. The NaCl concentration in the first filtration step is preferably between 1 and 3 M, more preferably between 1.5 and 2.5 M and most preferably it is 2 M.

In contrast, the prior art (Ge et al. (2006) Biotechnol. Bioeng. 95: 424-432) uses a pre-filtration step at room temperature without NaCl. Hence, contaminating proteins are removed less efficiently in this prior art method.

The purification method of the present disclosure preferably further involves the elution of the protein after the second filtration step at a temperature of 2°C to 8°C which greatly enhances the purification efficiency compared to prior art methods wherein the protein is eluted from the filter membrane at room temperature.

The term "filtration" is intended to describe a mechanical or physical process which is used for the separation of solids from liquids by interposing a medium through which only the fluid can pass and which retains solids which are larger than the pore size of the medium.

The term "inverse transition temperature" refers to the temperature above which the ELP fusion proteins aggregate and below which they remain soluble. The person skilled in the art knows that there is no fixed inverse transition temperature for a given construct, but that the inverse transition temperature depends on different factors such as the amino acid sequence and molecular weight of the ELP, the concentration of the ELP fusion protein and the composition of the solution. An overview of these factors is provided in Meyer and Chilkoti (2002) Protein Purification by Inverse Transition Cycling. In: Golemis and Adams (Eds.): Protein-Protein Interactions: A Molecular Cloning Manual. Cold Spring Harbor Laboratory Press, Cold Spring Harbor, pp. 329-343). The skilled person knows how to determine the inverse transition temperature for a given construct by routine methods.

In the two filtration steps a first and a second filter membrane are used, wherein the first and the second filter membrane can be the same or different. Preferably, the second filter membrane is different from the first filter membrane. The filter membrane in the first and second filtration step is preferably selected from polyethersulfone and cellulose acetate. Preferably, the filter membrane has a pore size of 0.2 µm. More preferably, the filter membrane in the first filtration step is polyethersulfone and the filter membrane in the second filtration step is cellulose acetate.

The second filtration step is preferably performed at room temperature.

Between the first and the second filtration step the filtered solution is preferably incubated for 1 to 10 minutes at a temperature between 18 and 25°C to precipitate the product. The skilled person knows that the exact incubation conditions may differ between proteins and can easily determine the conditions which are optimal for a given fusion protein.

After the second filtration step the filter membrane is washed for one to five times with 1 to 3 M NaCl, preferably at room temperature. After washing the filter membrane the protein is eluted from the membrane at a temperature between 2°C and 8°C, preferably at 4°C, using highly pure deionized water, preferably Millipore-Q water.

The solution which is subjected to the two filtration steps is prepared by a number of steps preceding the filtration steps.

First, an extract is prepared from plant material, preferably by grinding the plant material in liquid nitrogen. The extract may be prepared by adding any suitable buffer to the grinded plant material. Suitable buffers include 50 mM Tris, pH 8 without NaCl and phosphate buffer without NaCl, but with 0.1% Triton X-100. For purifying spider silk protein-ELP fusions phosphate buffer without NaCl, but with 0.1% Triton X-100 has been proven to be particularly suitable. For some proteins, including spider silk proteins, it may be necessary to heat the protein extract for a certain period, e.g. one hour, to a temperature between 80°C to 100°C.

After the extraction the extract is centrifuged at 60.000 to 80.000 g, preferably at 75.000 g, at a temperature between 2°C and 8°C, preferably 4°C, for 20 to 40 minutes, preferably for 30 minutes.

After the centrifugation NaCl, preferably in solid form, is added to the supernatant to a final NaCl concentration of 1 to 3 M, preferably 2 M, at a temperature between 2°C and 8°C, preferably 4°C. The resulting solution is then subjected to the first filtration step.

### EXAMPLES

### 1. Construction of the expression vectors and transformation

A nucleic acid sequence comprising the nucleic acid sequences of vector pCB301pHW401FLAG (see Figure 1a) starting from the nucleic acid sequence coding for the signal peptide and ending with the nucleic acid sequence coding for the N-terminal part of the intein having an NcoI restriction site at the 5' end and a BamHI restriction site at the 3' end was synthesized and inserted into the vector pRTRA 35S anti-Ag85B (BamHI)@19 (Floss et al. (2010) Journal of Biomedicine and Biotechnology 2010: 274346). Then the fragment from the promoter to the terminator was cut out from the resulting vector by HindIII and cloned into the binary vector pCB301-Kan resulting in the binary expression vector pCB301pHW401FLAG.

The binary vectors (derivatives of pCB301-Kan) were transformed into electro-competent agrobacteria (strain C58C1 Ti plasmid pGV2260) by electroporation (Gahrtz and Conrad (2009) Methods Mol. Biol. 483: 289-312; Floss and Conrad (2010) Expression of complete antibodies in transgenic plants. In: Kontermann, R. & S. Diibel (Eds): Antibody engineering (2nd edition). Springer-Verlag, Berlin-Heidelberg. pp. 490-502). Plasmid DNA was isolated from the resistant agrobacteria by classical methods and the recombinant agrobacteria have been selected by restriction analysis (Floss and Conrad (2010), *vide supra*).

*Nicotiana tabacum* plants were transformed by the leaf disc method essentially as described (Floss and Conrad (2010), *vide supra*). In brief, agrobacteria were grown and freshly cut leaf disks from tissue cultured leaves were incubated with the bacterial suspension. Leaf discs were grown on MS plates (3% sucrose) for two days in the dark at 24°C. Leaf disks were then transferred to solid MG medium plates with appropriate growth factors and antibiotics. Shoots emerging from the developing callus tissue were removed and grown on cefotaxime and the appropriate antibiotic. Rooted plantlets were later transferred into soil in the greenhouse and analysed.

By transformation with pCB301pHW401 FLAG-Intein 102 transgenic plants were produced. These plants were analyzed by Western blotting using the c-myc tag. Extraction, separation by SDS-PAGE, electroblotting and detection was performed as described in Conrad and Floss (2010) Expression of antibody fragments in transgenic plants. In: Kontermann, R. & S. Dübel (Eds): Antibody engineering (2nd edition). Springer-Verlag, Berlin-Heidelberg. pp. 377-386). Of the 102 plants analyzed 41 expressed the spider silk proteins.

### 2. Analysis of the transgenic plants

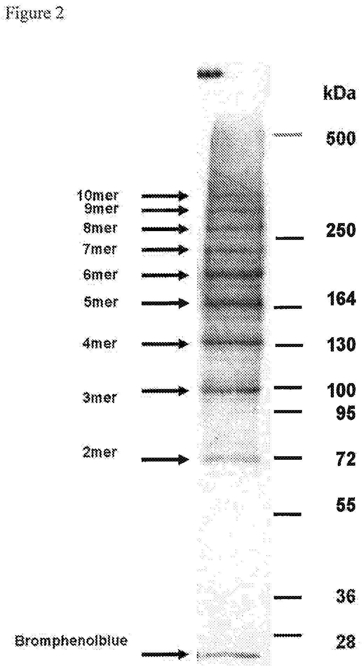

Leaves of pCB301pHW401 FLAG-Intein transgenic plants expressing the spider silk FLAG proteins were pooled and grinded with liquid nitrogen. The grinded frozen material was extracted with phosphate buffered saline containing 0.1% Triton X-100, pH 7.4 and clarified by centrifugation. The clear extract was mixed with an appropriate amount of 2xSDS probe buffer and applied to a 3-12% SDS-PAA gradient gel, separated, blotted and analyzed (see Figure 2).

Only multimers from dimers to 10mers are clearly visible. Larger multimers (up to a molecular weight of 500 kDa) are present, but not separated on this type of gel. Recombinant spider silk proteins of this size (≥ 250 kDa) have not been described so far.

To confirm that the production of multimers is due to the intein-mediated trans-splicing, a genetic control experiment was performed wherein strongly conserved amino acids from the inteins were mutated (see Figure 3a) and introduced into the vector pCB301pHW401 FLAG described in Example 1 resulting in the vector pCB301pHW401 FLAG-Inteinmut. Transgenic plants were produced using the vector with the mutated intein sequences and analyzed for the expression of the protein (see Figure 3b).

By transformation with pCB301pHW401 FLAG-Inteinmut 77 transgenic plants were produced. These plants were analyzed by Western blotting using the c-myc tag. Extraction, separation by SDS-PAGE, electroblotting and detection were performed as described in Conrad and Floss (2010) (*vide supra*). Of the 77 plants analyzed 41 expressed the spider silk proteins.

All plants analyzed only expressed the monomer including the extein and intein fragments. Protein splicing and protein fusions to polymers did not occur. This is the genetic proof that the occurrence of FLAG multimers is a function of inteins *in planta.*

### 3. Comparative Example: Purification of Spider silk proteins by Inverse Transition Cycling- Microfiltration

### a) Protein extraction

Deep-frozen leaf material from transgenic tobacco plants expressing MaSpI-ELP (Scheller et al. (2004) Transgenic Res. 13: 51-57), i.e. a naturally occurring spider silk protein fused to ELP, was grinded with liquid nitrogen. 250 ml phosphate buffer, pH 7.4, 0.1% Triton X-100 was heated to 90°C. 60 g grinded leaf material was added to the hot buffer by continuous stirring. The suspension was kept at a temperature of 95°C by continuous stirring for 1 hour. The temperature should not be below 80°C to prevent proteolysis.

### b) Membrane-based Inverse Transition Cycling

After cooling to 4°C, the suspension was centrifuged at 75.600 g and 4°C for 30 minutes. The cooled supernatant (5-8°C) was stirred with solid NaCl which was added to a final concentration of 2M until all salt was desolved. This ice-cold salt extract was pre-filtered at 4°C using a 0.22 µm polyethersulfone membrane. The filtered clear extract was pre-warmed to 18-20°C, kept at this temperature not longer then 2 minutes and filtered again using a 0.2 µm cellulose acetate membrane. The membrane was washed 2 times with 2M NaCl at room temperature and eluted with ice-cold Millipore water.

### c) Analysis of the purified proteins

Aliquots of the eluted spider silk proteins (MaSpI-ELP) were mixed with SDS probe buffer and analyzed by SDS PAGE and Western blotting (see Conrad and Floss, (2010), *vide supra*). Figure 4a shows highly enriched spider silk-ELP fusion proteins. The robustness of the method was proven by repeating this procedure three times yielding comparable results.

### 4. Comparative Example: Purification of neuraminidase by Inverse Transition Cycling- Microfiltration

### a) Construction of the expression vector and plant transformation

First, a polyhistidine affinity purification tag (6xHis) was added to the C-terminus of TBAG to create the vectors pRTRA-TBAG and pRTRA-TBAG-ELP (Floss et al. (2010) J. Biomed. Biotechnol. 2010: 1-15) comprising the constitutively expressing cauliflower mosaic virus (CaMV) 35S promoter, the legumin B4 (LeB4) signal peptide (SP), TBAG, a c-myc tag, 100 copies of ELP, and the KDEL ER retention signal. Next, the DNA sequence encoding NA 1 (SEQ ID No. 12) was PCR amplified using pPCR-N1 (GenBank accession AJ867075) as template and the primers according to SEQ ID Nos. 29 and 30; the amplicon was inserted into pRTRA-TBAG-ELP to replace the sequence DNA encoding TBAG to form the expression cassette pRTRA-35S-N1-ELP. The cassette was then *Hind*III digested and inserted into the pCB301-based shuttle vector pCB301-Kan (Floss et al. (2008) Plant Biotechnol. J. 6: 379-391; Xiang et al. (1999) Plant Mol. Biol. 40: 711-717).

The pCB301-Kan vector was electroporated into *Agrobacterium tumefaciens* strain C58C1 (pGV2260) (Deblaere et al. (1985) Nucleic Acids Res. 13: 4777-88) which was then used to transform the tobacco cultivar SNN *via* a leaf disk method (Horsch et al. (1985) Science 227: 1229-1231). Transgenic plants were cultured on Murashige and Skoog medium containing 50mg/L kanamycin and analysed by Western blot using a monoclonal antibody anti-c-myc antibody. Transgenic high-producer tobacco lines were selected and further propagated.

### b) Membrane-based Inverse Transition Cycling of ELP fusion proteins

Frozen leaf (150 g) was ground with mortar and pestle in liquid nitrogen and homogenized in 220 ml ice-cold 50mM Tris-HCl (pH 8.0). A Complete Protease Inhibitor Cocktail tablet (Roche, Germany) was added to the slurry, which was then cleared by centrifugation (75.600 g, 30min, 4°C) before NaCl was added to a final concentration of 2M. The cold extract with 2M NaCl was centrifuged again at 75,600 g for 30min at 4°C. The solution was then passed through a 0.22µm polyethersulfone membrane (Corning, USA) with the temperature maintained at 4°C, to produce a pre-treated extract. The pre-treated extract was warmed to room temperature and passed through a 0.2µm cellulose acetate membrane (Sartorius Stedim, Goettingen, Germany) using a vacuum pump (Vacuubrand, Germany). The membrane was washed twice with 2M NaCl at room temperature to remove contaminating proteins. Ice-cold Millipore-Q water was then passed through the filter to elute the protein-ELP fusions.

### c) SDS-PAGE and Western Blot analysis of the pruified proteins

The plant proteins obtained by the membrane-based inverse transition cycling were separated by reducing SDS-PAGE (10% polyacrylamide) and electrotransferred to nitrocellulose membranes. After blocking with 5% w/v fat-free milk powder dissolved in TBS (20mM Tris, 180mM NaCl, pH 7.8), the membranes were incubated for 2h at room temperature with a monoclonal anti-c-myc antibody. The presence of this antibody was detected by the addition of a 1:2,000 dilution of HRP-conjugated sheep anti-mouse IgG (Amersham, Germany), and the signal visualized using the ECL method (Amersham Biosciences). Each membrane was washed three times between each step with TBS containing 0.5% w/v fat-free milk, except for the penultimate (TBS only) and final (phosphate-buffered saline, PBS) washes. Antibodies were diluted in TBS, 5% w/v fat-free milk powder. The results of the Western Blot are shown in Figure 4b.

### d) N1-ELP activity assay

A fluorescence-based assay was used to measure neuraminidase (NA) activity, as described elsewhere (Potier et al. (1979) Anal. Biochem. 94: 287-96; Yongkiettrakul, et al. (2009) J. Virol. Methods 156: 44-51). The assay was performed by adding a 10µl aliquot of the N1-ELP sample to an equal volume of 32.5mM MES, 4mM CaC12, pH 6.5. The enzymatic reaction was then initiated by the addition of 30µl 2'-(4-methylumbelliferyl)-α-D-N-acetylneuraminic acid (Sigma, St. Louis, MO, USA). After incubation at 37°C for 30min, the reaction was stopped by the addition of 150µl 100µM glycine (pH 10.7) in 25% ethanol. The release of 4-methylumbelliferone (4-MU) was measured spectrophotometrically at 355 and 460nm, and quantification was based on a standard curve prepared with pure free 4-MU (Sigma, St. Louis, MO, USA). One unit of NA was defined as the quantity able to release 1µM 4-MU per min at 37°C. To determine the efficiency of N1-ELP purification based on its NA activity, total protein was precipitated from the initial plant extract with ammonium sulfate, and pelleted by centrifugation (38,000 g, 30min, 4°C). The pellet was re-solubilized in PBS, and the sample treated as above to determine NA activity. The % recovery of N1-ELP based on NA activity was defined as the ratio between the NA activity before and after purification.

**Table 1 shows the enzymatic activity of N1-ELP in the raw extract and after purification by membrane-based inverse transition cycling**

| Status of N1-ELP | Total protein (mg) | Total activity (U) | Recovery rate (%) | Specific activity (U mg⁻¹) |
|---|---|---|---|---|
| Raw extract | 3521.9 | 736.5 | 100 | 0.21 |
| Purified N1-ELP | 20.3 | 681.1 | 92.5 | 33.6 |

## Claims

1. Method of producing a recombinant polymeric protein in transgenic plants or plant cells, comprising the steps of:
a) introducing by transformation into the plant or plant cell at least one recombinant nucleic acid molecule comprising the following elements in 5' to 3' orientation:
- a promoter functional in plant cells;
- operatively linked thereto a nucleic acid sequence coding for the C-terminal part of an intein;
- a nucleic acid sequence coding for a monomer of the polymeric protein;
- a nucleic acid sequence coding for the N-terminal part of an intein; and
- optionally, operatively linked thereto a terminator sequence functional in plant cells;
wherein the recombinant nucleic acid molecule further comprises a nucleic acid sequence coding for 10 to 100 repeats of the pentapeptide Val-Pro-Gly-Xaa-Gly (ELP), wherein Xaa can be any amino acid except proline, which nucleic acid sequence is located 5' or 3' of or within the nucleic acid sequence coding for the monomer of the polymeric protein, wherein the nucleic acid sequence coding for the ELP is positioned next to the nucleic acid sequence coding for the monomer of the polymeric protein so that a single open reading frame is created;
b) optionally, regenerating plants from the transformed plant cells; and
c) isolating the recombinant polymeric protein from the transgenic plant or plant cells.

2. Method according to claim 1, wherein one recombinant nucleic acid molecule as described in claim 1 is introduced into the plant or plant cell and wherein the C-terminal part of the intein and the N-terminal part of the intein are derived from the same intein.
RN:LA

3. Method according to claim 1, wherein two recombinant nucleic acid molecules as described in claim 1 are introduced into the plant or plant cell, wherein the first recombinant nucleic acid molecule comprises the C-terminal part of a first intein and the N-terminal part of a second intein and wherein the second recombinant nucleic acid molecule comprises the C-terminal part of said second intein and the N-terminal part of said first intein.

4. Method according to any of the preceding claims, wherein the recombinant polymeric protein is selected from the group consisting of spider silk proteins, fibroins, seed storage proteins and collagen.

5. Method according to any of the preceding claims, wherein the step of isolating the recombinant polymeric protein comprises a first and a second filtration step, wherein the first filtration step is performed in the presence of NaCl and at a temperature between 4°C and a temperature which is at least 5°C below the inverse transition temperature of the fusion protein.

6. Method according to claim 5, wherein after the second filtration step the fusion protein is eluted at a temperature of 2°C to 8°C.

7. Recombinant polymeric protein obtainable by the method of any of claims 1 to 6, wherein the recombinant polymeric protein has a molecular weight of at least 180 kD and a structure selected from the group of
a) (monomer-ELP)ₓ,
b) (ELP-monomer)ₓ or
c) monomerₓ, wherein ELP is located within the monomer,
wherein x is 2 to 25 and wherein ELP means 10 to 100 repeats of the pentapeptide Val-Pro-Gly-Xaa-Gly (ELP), wherein Xaa can be any amino acid except proline.

8. Recombinant polymeric protein according to claim 7, being a spider silk protein.

9. Use of the spider silk protein according to claim 8 for producing synthetic threads, films and/or membranes.

10. Recombinant nucleic acid molecule, comprising the following elements in 5' to 3' orientation:
- a promoter functional in plant cells;
- operatively linked thereto a nucleic acid sequence coding for the C-terminal part of an intein;
- a nucleic acid sequence coding for a monomer of a polymeric protein;
- a nucleic acid sequence coding for the N-terminal part of an intein; and
- optionally, operatively linked thereto a terminator sequence functional in plant cells;
wherein the recombinant nucleic acid molecule further comprises a nucleic acid sequence coding for 10 to 100 repeats of the pentapeptide Val-Pro-Gly-Xaa-Gly (ELP), wherein Xaa can be any amino acid except proline, which nucleic acid sequence is located 5' or 3' of or within the nucleic acid sequence coding for the monomer of the polymeric high molecular weight protein.

11. Recombinant nucleic acid molecule according to claim 10, wherein the polymeric protein is selected from the group consisting of spider silk proteins, fibroins, seed storage proteins and collagen.

12. Transgenic plant comprising a recombinant nucleic acid molecule according to claim 10 or 11 as well as transgenic parts of this plant comprising a recombinant nucleic acid molecule according to claim 10 or 11, transgenic harvest products of this plant comprising a recombinant nucleic acid molecule according to claim 10 or 11 and transgenic propagating material of this plant comprising a recombinant nucleic acid molecule according to claim 10 or 11, such as protoplasts, plant cells, calli, seeds, tubers, cuttings, and the transgenic progeny of this plant comprising a recombinant nucleic acid molecule according to claim 10 or 11.

## Patentansprüche

1. Verfahren zur Herstellung eines rekombinanten polymeren Proteins in transgenen Pflanzen oder Pflanzenzellen, umfassend die Schritte:
a) Einführen durch Transformation in die Pflanze oder Pflanzenzelle mindestens eines rekombinanten Nukleinsäuremoleküls umfassend die folgenden Elemente in 5' nach 3' Orientierung:
- ein Promotor, der in Pflanzenzellen funktionsfähig ist;
- operativ daran gebunden eine Nukleinsäuresequenz, die für den C-terminalen Teil eines Inteins kodiert;
- eine Nukleinsäuresequenz, die für ein Monomer des polymeren Proteins kodiert;
- eine Nukleinsäuresequenz, die für den N-terminalen Teil eines Inteins kodiert; und
- ggf. operativ daran gebunden eine Terminatorsequenz, die in Pflanzenzellen funktionsfähig ist;
wobei das rekombinante Nukleinsäuremolekül weiter umfasst eine Nukleinsäuresequenz, die für 10 bis 100 Wiederholungen des Pentapeptids Val-Pro-Gly-Xaa-Gly (ELP) kodiert, wobei Xaa jede Aminosäure außer Prolin sein kann, wobei sich die Nukleinsäuresequenz 5' oder 3' von oder innerhalb der Nukleinsäuresequenz, die für das Monomer des polymeren Proteins kodiert, befindet, wobei die Nukleinsäuresequenz, die für das ELP kodiert, neben der Nukleinsäuresequenz positioniert ist, die für das Monomer des polymeren Proteins kodiert, so dass ein einzelnes offenes Leseraster gebildet wird;
b) ggf. Regenerieren von Pflanzen aus den transformierten Pflanzenzellen; und
c) Isolieren des rekombinanten polymeren Proteins aus der transgenen Pflanze oder den Pflanzenzellen.

2. Verfahren nach Anspruch 1, wobei ein rekombinantes Nukleinsäuremolekül wie in Anspruch 1 in die Pflanze oder Pflanzenzelle eingeführt wird, und wobei der C-terminale Teil des Inteins und der N-terminale Teil des Inteins aus demselben Intein stammen.

3. Verfahren nach Anspruch 1, wobei zwei rekombinante Nukleinsäuremoleküle wie in Anspruch 1 beschrieben in die Pflanze oder Pflanzenzelle eingeführt werden, wobei das erste rekombinante Nukleinsäuremolekül den C-terminalen Teil eines ersten Inteins und den N-terminalen Teil eines zweiten Inteins umfasst, und wobei das zweite rekombinante Nukleinsäuremolekül den C-terminalen Teil des zweiten Inteins und den N-terminalen Teil des ersten Inteins umfasst.

4. Verfahren nach einem der vorhergehenden Ansprüche, wobei das rekombinante polymere Protein ausgewählt ist aus der Gruppe bestehend aus Spinnenseidenproteinen, Fibroinen, Samenspeicherproteinen und Kollagen.

5. Verfahren nach einem der vorhergehenden Ansprüche, wobei der Schritt des Isolierens des rekombinanten polymeren Proteins einen ersten und einen zweiten Filtrationsschritt umfasst, wobei der erste Filtrationsschritt in Gegenwart von NaCl und bei einer Temperatur zwischen 4°C und einer Temperatur, die mindestens 5°C unter der inversen Übergangstemperatur des Fusionsproteins liegt, durchgeführt wird.

6. Verfahren nach Anspruch 5, wobei das Fusionsprotein nach dem zweiten Filtrationsschritt bei einer Temperatur von 2°C bis 8°C eluiert wird.

7. Rekombinantes polymeres Protein, das durch das Verfahren nach einem der Ansprüche 1 bis 6 erhältlich ist, wobei das rekombinante polymere Protein aufweist ein Molekulargewicht von mindestens 180 kD und eine Struktur ausgewählt aus der Gruppe bestehend aus
a) (Monomer-ELP)ₓ,
b) (ELP-Monomer)ₓ, oder
c) Monomerₓ, wobei sich ELP innerhalb des Monomers befindet,
wobei x 2 bis 25 ist und wobei ELP 10 bis 100 Wiederholungen des Pentapeptids Val-Pro-Gly-Xaa-Gly (ELP) bedeutet, wobei Xaa jede Aminosäure außer Prolin sein kann.

8. Rekombinantes polymeres Protein nach Anspruch 7, das ein Spinnenseidenprotein ist.

9. Verwendung des Spinnenseidenproteins nach Anspruch 8 zur Herstellung von synthetischen Fäden, Filmen und/oder Membranen.

10. Rekombinantes Nukleinsäuremolekül umfassend die folgenden Elemente in 5' nach 3' Orientierung:
- ein Promotor, der in Pflanzenzellen funktionsfähig ist;
- operativ daran gebunden eine Nukleinsäuresequenz, die für den C-terminalen Teil eines Inteins kodiert;
- eine Nukleinsäuresequenz, die für ein Monomer eines polymeren Proteins kodiert;
- eine Nukleinsäuresequenz, die für den N-terminalen Teil eines Inteins kodiert; und
- ggf. operativ daran gebunden eine Terminatorsequenz, die in Pflanzenzell funktionsfähig ist;
wobei das rekombinante Nukleinsäuremolekül weiter umfasst eine Nukleinsäuresequenz, die für 10 bis 100 Wiederholungen des Pentapeptids Val-Pro-Gly-Xaa-Gly (ELP) kodiert, wobei Xaa jede Aminosäure außer Prolin sein kann, wobei sich die Nukleinsäuresequenz 5' oder 3' von oder innerhalb der Nukleinsäuresequenz befindet, die für das Monomer des polymeren Proteins mit hohem Molekulargewicht kodiert.

11. Rekombinantes Nukleinsäuremolekül nach Anspruch 10, wobei das polymere Protein ausgewählt ist aus der Gruppe bestehend aus Spinnenseidenproteinen, Fibroinen, Samenspeicherproteinen und Kollagen.

12. Transgene Pflanze umfassend ein rekombinantes Nukleinsäuremolekül nach Anspruch 10 oder 11 sowie transgene Teile dieser Pflanze umfassend ein rekombinantes Nukleinsäuremolekül nach Anspruch 10 oder 11, transgene Ernteprodukte dieser Pflanze umfassend ein rekombinantes Nukleinsäuremolekül nach Anspruch 10 oder 11 und transgenes Fortpflanzungsmaterial dieser Pflanze umfassend eine rekombinantes Nukleinsäuremolekül nach Anspruch 10 oder 11, wie Protoplasten, Pflanzenzellen, Kalli, Samen, Knollen , Stecklinge, und die transgenen Nachkommen dieser Pflanze umfassend ein rekombinantes Nukleinsäuremolekül nach Anspruch 10 oder 11.

## Revendications

1. Procédé de production d'une protéine polymère recombinante dans des plantes ou cellules de plante transgéniques, comprenant les étapes de :
a) introduction par transformation dans la plante ou cellule de plante d'au moins une molécule d'acide nucléique recombinante comprenant les éléments suivants en orientation 5' à 3' :
- un promoteur fonctionnel dans des cellules de plante ;
- en liaison opérationnelle avec celui-ci, une séquence d'acide nucléique codant pour la partie C-terminale d'une intéine ;
- une séquence d'acide nucléique codant pour un monomère de la protéine polymère ;
- une séquence d'acide nucléique codant pour la partie N-terminale d'une intéine ; et
- facultativement, en liaison opérationnelle avec celle-ci, une séquence de terminaison fonctionnelle dans des cellules de plante ;
sachant que la molécule d'acide nucléique recombinante comprend en outre une séquence d'acide nucléique codant pour 10 à 100 répétitions du pentapeptide Val-Pro-Gly-Xaa-Gly (ELP), sachant que Xaa peut être n'importe quel acide aminé à l'exception de la proline, laquelle séquence d'acide nucléique est située en 5' ou 3' de ou à l'intérieur de la séquence d'acide nucléique codant pour le monomère de la protéine polymère, sachant que la séquence d'acide nucléique codant pour l'ELP est positionnée à côté de la séquence d'acide nucléique codant pour le monomère de la protéine polymère de sorte qu'un cadre de lecture ouvert unique soit créé ;
b) facultativement, régénération de plantes à partir des cellules de plante transformées ; et
c) isolation de la protéine polymère recombinante de la plante ou des cellules de plante transgéniques.

2. Procédé selon la revendication 1, sachant qu'une molécule d'acide nucléique recombinante telle que décrite dans la revendication 1 est introduite dans la plante ou cellule de plante et sachant que la partie C-terminale de l'intéine et la partie N-terminale de l'intéine sont dérivées de la même intéine.

3. Procédé selon la revendication 1, sachant que deux molécules d'acide nucléique recombinantes telles que décrites dans la revendication 1 sont introduites dans la plante ou cellule de plante, sachant que la première molécule d'acide nucléique recombinante comprend la partie C-terminale d'une première intéine et la partie N-terminale d'une deuxième intéine et sachant que la deuxième molécule d'acide nucléique recombinante comprend la partie C-terminale de ladite deuxième intéine et la partie N-terminale de ladite première intéine.

4. Procédé selon l'une quelconque des revendications précédentes, sachant que la protéine polymère recombinante est sélectionnée dans le groupe constitué par les protéines de soie d'araignée, les fibroïnes, les protéines de stockage de graines et le collagène.

5. Procédé selon l'une quelconque des revendications précédentes, sachant que l'étape d'isolation de la protéine polymère recombinante comprend une première et une deuxième étape de filtration, sachant que la première étape de filtration est effectuée en présence de NaCl et à une température entre 4°C et une température qui est d'au moins 5°C sous la température de transition inverse de la protéine de fusion.

6. Procédé selon la revendication 5, sachant qu'après la deuxième étape de filtration, la protéine de fusion est éluée à une température de 2°C à 8°C.

7. Protéine polymère recombinante pouvant être obtenue par le procédé de l'une quelconque des revendications 1 à 6, sachant que la protéine polymère recombinante a un poids moléculaire d'au moins 180 kD et une structure sélectionnée dans le groupe de
a) (monomère-ELP)ₓ,
b) (ELP-monomère)ₓ ou
c) monomèreₓ, sachant qu'ELP est situé à l'intérieur du monomère,
sachant que x est 2 à 25 et sachant qu'ELP signifie 10 à 100 répétitions du pentapeptide Val-Pro-Gly-Xaa-Gly (ELP), sachant que Xaa peut être n'importe quel acide aminé à l'exception de la proline.

8. Protéine polymère recombinante selon la revendication 7, laquelle est une protéine de soie d'araignée.

9. Utilisation de la protéine de soie d'araignée selon la revendication 8 pour la production de fils, films et/ou membranes synthétiques.

10. Molécule d'acide nucléique recombinante, comprenant les éléments suivants en orientation 5' à 3' :
- un promoteur fonctionnel dans des cellules de plante ;
- en liaison opérationnelle avec celui-ci, une séquence d'acide nucléique codant pour la partie C-terminale d'une intéine ;
- une séquence d'acide nucléique codant pour un monomère d'une protéine polymère ;
- une séquence d'acide nucléique codant pour la partie N-terminale d'une intéine ; et
- facultativement, en liaison opérationnelle avec celle-ci, une séquence de terminaison fonctionnelle dans des cellules de plante ;
sachant que la molécule d'acide nucléique recombinante comprend en outre une séquence d'acide nucléique codant pour 10 à 100 répétitions du pentapeptide Val-Pro-Gly-Xaa-Gly (ELP), sachant que Xaa peut être n'importe quel acide aminé à l'exception de la proline, laquelle séquence d'acide nucléique est située en 5' ou 3' de ou à l'intérieur de la séquence d'acide nucléique codant pour le monomère de la protéine polymère de poids moléculaire élevé.

11. Molécule d'acide nucléique recombinante selon la revendication 10, sachant que la protéine polymère est sélectionnée dans le groupe constitué par les protéines de soie d'araignée, les fibroïnes, les protéines de stockage de graines et le collagène.

12. Plante transgénique comprenant une molécule d'acide nucléique recombinante selon la revendication 10 ou 11 ainsi que parties transgéniques de cette plante comprenant une molécule d'acide nucléique recombinante selon la revendication 10 ou 11, produits de récolte transgéniques de cette plante comprenant une molécule d'acide nucléique recombinante selon la revendication 10 ou 11 et matériau de propagation transgénique de cette plante comprenant une molécule d'acide nucléique recombinante selon la revendication 10 ou 11, tels que protoplastes, cellules de plante, calli, graines, tubercules, boutures, et la progéniture transgénique de cette plante comprenant une molécule d'acide nucléique recombinante selon la revendication 10 ou 11.


### SEQUENCE LISTING

<110> Leibniz-Institut für Pflanzengentik und Kulturpflanzenforschung
<120> Method of producing and purifying polymeric proteins in transgenic plants
<130> I 8294/RN
<160> 32
<170> PatentIn version 3.5
<210> 1
   <211> 197
   <212> PRT
   <213> artificial
<220>
   <223> FLAG amino acid sequence
<400> 1
   Gly Pro Gly Gly Tyr Gly Pro Gly Gly Ala Gly Pro Gly Gly Tyr Gly
   1               5                   10                  15
   Pro Gly Gly Ala Gly Pro Gly Gly Tyr Gly Pro Gly Gly Ala Gly Pro
               20                  25                  30
   Gly Gly Tyr Gly Pro Gly Gly Thr Gly Pro Gly Gly Tyr Gly Pro Gly
           35                  40                  45
   Gly Thr Gly Pro Gly Gly Val Gly Pro Gly Gly Ala Gly Pro Gly Gly
       50                  55                  60
   Tyr Gly Pro Gly Gly Ala Gly Pro Gly Gly Ala Gly Pro Gly Gly Ala
   65                  70                  75                  80
   Gly Pro Gly Gly Ala Gly Pro Gly Gly Ala Gly Pro Gly Gly Ala Gly
                   85                  90                  95
   Pro Gly Gly Tyr Gly Pro Gly Gly Ser Gly Pro Gly Gly Ala Thr Ile
               100                 105                 110
   Ile Glu Asp Leu Asp Ile Thr Ile Asp Gly Ala Asp Gly Pro Pro Ile
           115                 120                 125
   Thr Ile Ser Glu Glu Leu Thr Ile Ser Gly Ala Gly Gly Ser Gly Pro
       130                 135                 140
   Gly Gly Val Gly Pro Gly Gly Ser Gly Pro Gly Gly Val Gly Pro Gly
   145                 150                 155                 160
   Ser Phe Gly Pro Gly Gly Val Gly Pro Gly Gly Ser Gly Pro Gly Gly
                   165                 170                 175
   Val Gly Ser Gly Gly Ser Gly Gln Gly Gly Val Arg Pro Ser Gly Ser
                   180                 185                 190
   Gly Pro Gly Gly Val
           195
<210> 2
   <211> 635
   <212> PRT
   <213> artificial
<220>
   <223> SO1 amino acid sequence
<400> 2
   Gly Gln Gly Gly Tyr Gly Gly Leu Gly Gly Gln Gly Ala Gly Gln Gly
   1               5                   10                  15
   Gly Tyr Gly Gly Leu Gly Gly Gln Gly Ala Gly Gln Gly Ala Gly Ala
               20                  25                  30
   Ala Ala Ala Ala Ala Gly Gly Ala Gly Gln Gly Gly Tyr Gly Gly Leu
           35                  40                  45
   Gly Ser Gln Gly Ala Gly Arg Gly Gly Gln Gly Ala Gly Ala Ala Ala
       50                  55                  60
   Ala Ala Ala Gly Gly Ala Gly Gln Gly Gly Tyr Gly Gly Leu Gly Ser
   65                  70                  75                  80
   Gln Gly Ala Gly Arg Gly Gly Leu Gly Gly Gln Gly Ala Gly Ala Ala
                   85                  90                  95
   Ala Ala Ala Ala Gly Gly Ala Gly Gln Gly Gly Tyr Gly Gly Leu Gly
               100                 105                 110
   Ser Gln Gly Ala Gly Arg Gly Gly Gln Gly Ala Gly Ala Ala Ala Ala
           115                 120                 125
   Ala Ala Gly Gly Ala Gly Gln Gly Gly Tyr Gly Gly Leu Gly Ser Gln
       130                 135                 140
   Gly Ala Gly Arg Gly Gly Leu Gly Gly Gln Gly Ala Gly Ala Ala Ala
   145                 150                 155                 160
   Ala Ala Ala Gly Gly Ala Gly Gln Gly Gly Tyr Gly Gly Leu Gly Gly
                   165                 170                 175
   Gln Gly Ala Gly Gln Gly Gly Tyr Gly Gly Leu Gly Ser Gln Gly Ala
               180                 185                 190
   Gly Arg Gly Gly Leu Gly Gly Gln Gly Ala Gly Ala Ala Ala Ala Ala
           195                 200                 205
   Ala Gly Gly Ala Gly Gln Gly Gly Leu Gly Gly Gln Gly Ala Gly Gln
       210                 215                 220
   Gly Ala Gly Ala Ala Ala Ala Ala Ala Gly Gly Ala Gly Gln Gly Gly
   225                 230                 235                 240
   Tyr Gly Gly Leu Gly Ser Gln Gly Ala Gly Arg Gly Gly Gln Gly Ala
                   245                 250                 255
   Gly Ala Ala Ala Ala Ala Ala Gly Gly Ala Gly Gln Gly Gly Tyr Gly
               260                 265                 270
   Gly Leu Gly Gly Gln Gly Ala Gly Gln Gly Gly Tyr Gly Gly Leu Gly
           275                 280                 285
   Ser Gln Gly Ala Gly Arg Gly Gly Leu Gly Gly Gln Gly Ala Gly Ala
       290                 295                 300
   Ala Ala Ala Ala Ala Gly Gly Ala Gly Gln Gly Gly Leu Gly Gly Gln
   305                 310                 315                 320
   Gly Ala Gly Gln Gly Ala Gly Ala Ala Ala Ala Ala Ala Gly Gly Ala
                   325                 330                 335
   Gly Gln Gly Gly Tyr Gly Gly Leu Gly Ser Gln Gly Ala Gly Arg Gly
               340                 345                 350
   Gly Leu Gly Gly Gln Gly Ala Gly Ala Ala Ala Ala Ala Ala Gly Gly
           355                 360                 365
   Ala Gly Gln Gly Gly Tyr Gly Gly Leu Gly Ser Gln Gly Ala Gly Arg
       370                 375                 380
   Gly Gly Gln Gly Ala Gly Ala Ala Ala Ala Ala Ala Gly Gly Ala Gly
   385                 390                 395                 400
   Gln Gly Gly Tyr Gly Gly Leu Gly Ser Gln Gly Ala Gly Arg Gly Gly
                   405                 410                 415
   Leu Gly Gly Gln Gly Ala Gly Ala Ala Ala Ala Ala Ala Gly Gly Ala
               420                 425                 430
   Gly Gln Gly Gly Tyr Gly Gly Leu Gly Ser Gln Gly Ala Gly Arg Gly
           435                 440                 445
   Gly Gln Gly Ala Gly Ala Ala Ala Ala Ala Ala Gly Gly Ala Gly Gln
       450                 455                 460
   Gly Gly Tyr Gly Gly Leu Gly Ser Gln Gly Ala Gly Arg Gly Gly Gln
   465                 470                 475                 480
   Gly Ala Gly Ala Ala Ala Ala Ala Ala Gly Gly Ala Gly Gln Gly Gly
   485 490 495
   Tyr Gly Gly Leu Gly Gly Gln Gly Ala Gly Gln Gly Gly Tyr Gly Gly
   500 505 510
   Leu Gly Ser Gln Gly Ala Gly Arg Gly Gly Leu Gly Gly Gln Gly Ala
   515 520 525
   Gly Ala Ala Ala Ala Ala Ala Gly Gly Ala Gly Gln Gly Gly Leu Gly
   530 535 540
   Gly Gln Gly Ala Gly Gln Gly Ala Gly Ala Ala Ala Ala Ala Ala Gly
   545 550 555 560
   Gly Ala Gly Gln Gly Gly Tyr Gly Gly Leu Gly Ser Gln Gly Ala Gly
   565 570 575
   Arg Gly Gly Gln Gly Ala Gly Ala Ala Ala Ala Ala Ala Gly Gly Ala
   580 585 590
   Gly Gln Gly Gly Tyr Gly Gly Leu Gly Ser Gln Gly Ala Gly Arg Gly
   595 600 605
   Gly Leu Gly Gly Gln Gly Ala Gly Ala Ala Ala Ala Ala Ala Gly Gly
   610 615 620
   Ala Gly Gly Gln Ala Ala Ala Glu Gln Lys Leu
   625 630 635
<210> 3
   <211> 431
   <212> PRT
   <213> Nephila clavipes
<400> 3
   Gly Gly Leu Gly Ile Gln Gly Ser Gly Arg Gly Gly Leu Gly Gly Gln
   1               5                   10                  15
   Gly Ala Val Ala Ala Ala Ala Ala Ala Ala Gly Gly Ala Val Gln Val
               20                  25                  30
   Val Leu Gly Gly Gln Gly Ala Gly Gln Gly Ala Gly Ala Ala Ala Ala
           35                  40                  45
   Ala Ala Gly Gly Ala Gly Gln Gly Gly Tyr Gly Gly Leu Gly Ser Gln
       50                  55                  60
   Gly Ala Gly Arg Gly Gly Gln Gly Ala Gly Ala Arg Thr Ala Ala Ala
   65                  70                  75                  80
   Val Gly Ala Gly Gln Gly Gly Tyr Gly Gly Gln Gly Ala Gly Gln Gly
                   85                  90                  95
   Gly Tyr Gly Gly Leu Gly Ser Gln Gly Ala Gly Arg Gly Gly Leu Gly
   100 105 110
   Gly Gln Gly Ala Gly Ala Ala Ala Ala Ala Ala Ala Gly Ser Ala Glu
   115 120 125
   Gln Gly Leu Gly Gly Gln Gly Ala Gly Gln Gly Ala Gly Ala Ala Ala
   130 135 140
   Ala Ala Ala Gly Gly Ala Gly Gln Gly Gly Tyr Gly Gly Leu Gly Ser
   145 150 155 160
   Gln Gly Ala Gly Arg Gly Gly Leu Gly Gly Gln Gly Ala Gly Ala Ala
   165 170 175
   Ala Ala Ala Ala Gly Gly Ala Gly Gln Gly Gly Tyr Gly Gly Leu Gly
   180 185 190
   Gly Gln Gly Ala Gly Gln Gly Gly Tyr Gly Gly Leu Gly Ser Gln Gly
   195 200 205
   Ser Gly Arg Gly Gly Leu Gly Gly Gln Gly Ala Gly Ala Ala Ala Ala
   210 215 220
   Ala Ala Gly Gly Ala Gly Gln Gly Gly Leu Gly Gly Gln Gly Ala Gly
   225 230 235 240
   Gln Gly Ala Gly Ala Ala Ala Ala Ala Ala Gly Gly Val Arg Gln Gly
   245 250 255
   Gly Tyr Gly Gly Leu Gly Ser Gln Gly Ala Gly Arg Gly Gly Gln Gly
   260 265 270
   Ala Gly Ala Ala Ala Ala Ala Ala Gly Gly Ala Gly Gln Gly Gly Tyr
   275 280 285
   Gly Gly Leu Gly Gly Gln Gly Val Gly Arg Gly Gly Leu Gly Gly Gln
   290 295 300
   Gly Ala Gly Ala Ala Ala Ala Gly Gly Ala Gly Gln Gly Gly Tyr Gly
   305 310 315 320
   Gly Val Gly Ser Gly Ala Ser Ala Ala Ser Ala Ala Ala Ser Arg Leu
   325 330 335
   Ser Ser Pro Gln Ala Ser Ser Arg Val Ser Ser Ala Val Ser Asn Leu
   340 345 350
   Val Ala Ser Gly Pro Thr Asn Ser Ala Ala Leu Ser Ser Thr Ile Ser
   355 360 365
   Asn Val Val Ser Gln Ile Gly Ala Ser Asn Pro Gly Leu Ser Gly Cys
   370 375 380
   Asp Val Leu Ile Gln Ala Leu Leu Glu Val Val Ser Ala Leu Ile His
   385 390 395 400
   Ile Leu Gly Ser Ser Ser Ile Gly Gln Val Asn Tyr Gly Ser Ala Gly
   405 410 415
   Gln Ala Thr Gln Ile Val Gly Gln Ser Val Tyr Gln Ala Leu Gly
   420 425 430
<210> 4
   <211> 276
   <212> PRT
   <213> Nephila clavipes
<400> 4
   Met Ser Trp Ser Thr Leu Ala Leu Ala Ile Leu Ala Val Leu Ser Thr
   1               5                   10                  15
   Gln Ser Ile Tyr Ala Ser Ala Gln Ala Arg Ser Pro Trp Ser Asp Thr
               20                  25                  30
   Ala Thr Ala Asp Ala Phe Ile Gln Asn Phe Leu Ala Ala Val Ser Gly
           35                  40                  45
   Ser Gly Ala Phe Thr Ser Asp Gln Leu Asp Asp Met Ser Thr Ile Gly
       50                  55                  60
   Asp Thr Ile Met Ser Ala Met Asp Lys Met Ala Arg Ser Asn Lys Ser
   65                  70                  75                  80
   Ser Gln His Lys Leu Gln Ala Leu Asn Met Ala Phe Ala Ser Ser Met
                   85                  90                  95
   Ala Glu Ile Ala Ala Val Glu Gln Gly Gly Met Ser Met Ala Val Lys
               100                 105                 110
   Thr Asn Ala Ile Val Asp Gly Leu Asn Ser Ala Phe Tyr Met Thr Thr
           115                 120                 125
   Gly Ala Ala Asn Pro Gln Phe Val Asn Glu Met Arg Ser Leu Ile Ser
       130                 135                 140
   Met Ile Ser Ala Ala Ser Ala Asn Glu Val Ser Tyr Gly Gly Gly Ala
   145                 150                 155                 160
   Ser Ala Ala Ala Ala Ala Ala Gly Gly Tyr Gly Gln Gly Pro Ser Gly
                   165                 170                 175
   Tyr Gly Pro Gly Ala Ser Ala Ser Ser Ala Ala Ala Ala Ala Ala Pro
               180                 185                 190
   Ser Gly Tyr Gly Pro Ser Gln Gln Gly Pro Ser Gly Ser Ala Ala Ala
           195                 200                 205
   Ala Ala Ala Ala Ala Pro Ser Gly Tyr Gly Pro Arg Gln Gln Ala Pro
       210                 215                 220
   Ser Gly Pro Ala Ala Ala Ala Ala Ala Ala Gly Ala Gly Gly Tyr Gly
   225                 230                 235                 240
   Pro Ala Gln Arg Gly Pro Ser Val Tyr Gly Pro Ser Gly Pro Gly Ala
                   245                 250                 255
   Ala Ala Ala Ala Ala Ala Gly Ala Gly Pro Gly Gly Tyr Gly Pro Gly
               260                 265                 270
   Gln Gln Gly Pro
           275
<210> 5
   <211> 444
   <212> PRT
   <213> Triticum aestivum
<400> 5
   Met Ala Lys Arg Leu Val Leu Phe Ala Ala Val Val Ile Ala Leu Val
   1               5                   10                  15
   Ala Leu Thr Thr Ala Ser Gln Val Ala Arg Gln Tyr Glu Gln Thr Val
               20                  25                  30
   Val Pro Pro Lys Gly Gly Ser Phe Tyr Pro Gly Glu Thr Thr Pro Leu
           35                  40                  45
   Pro Gln Leu Gln Gln Gly Ile Phe Trp Gly Thr Ser Ser Gln Thr Val
       50                  55                  60
   Gln Gly Tyr Tyr Pro Gly Val Thr Ser Pro Arg Gln Gly Ser Tyr Tyr
   65                  70                  75                  80
   Pro Gly Gln Ala Ser Pro Gln Gln Pro Gly Gln Gly Gln Gln Pro Gly
                   85                  90                  95
   Lys Trp Gln Glu Pro Gly Gln Gly Gln Gln Trp Tyr Tyr Pro Thr Ser
               100                 105                 110
   Leu Gln Gln Pro Gly Gln Gly Gln Gln Ser Pro Ala His Arg Thr Lys
           115                 120                 125
   Ala Thr Thr Ser Thr Arg Ala Thr Thr Arg Thr Arg Ala Thr Thr Arg
       130                 135                 140
   Thr Met Ala Thr Arg Val Leu Ser Asn Phe Ser Thr Thr Ala Arg Thr
   145                 150                 155                 160
   Arg Ala Thr Thr Lys Thr Met Ala Thr Ile Arg Thr Arg Ala Thr Arg
                   165                 170                 175
   Ala Leu Pro Asn Phe Ser Thr Thr Ala Arg Thr Arg Ala Thr Arg Ala
               180                 185                 190
   Leu Pro Ser Phe Ser Ala Ala Ala Arg Thr Arg Ala Thr Arg Ala Leu
           195                 200                 205
   Pro Ser Phe Ser Ala Ala Ala Arg Thr Arg Ala Thr Arg Ala Leu Pro
       210                 215                 220
   Ser Phe Ser Ala Ala Ala Arg Thr Arg Ala Thr Thr Ala Arg Thr Arg
   225                 230                 235                 240
   Ala Thr Thr Arg Thr Arg Ala Thr Arg Ile Leu Ser Asn Phe Ser Gly
                   245                 250                 255
   Ala Ala Arg Thr Arg Ala Thr Ala Arg Thr Arg Ala Thr Arg Val Leu
               260                 265                 270
   Pro Asn Phe Ser Pro Ala Ala Arg Thr Arg Ala Thr Arg Ala Leu Pro
           275                 280                 285
   Ser Phe Ser Val Pro Ala Arg Thr Arg Thr Ala Arg Thr Lys Ala Thr
       290                 295                 300
   Thr Arg Thr Arg Ala Thr Ser Arg Thr Arg Glu Thr Thr Arg Thr Arg
   305                 310                 315                 320
   Ala Thr Arg Val Leu Ser Asn Phe Ser Thr Ala Ala Arg Thr Arg Ala
                   325                 330                 335
   Thr Thr Arg Thr Arg Ala Thr Arg Val Leu Pro Asn Phe Ser Ala Ala
               340                 345                 350
   Ala Arg Thr Arg Ala Thr Thr Arg Thr Arg Ala Thr Arg Ala Leu Pro
           355                 360                 365
   Asn Val Pro Ala Ala Val Arg Thr Ser Ala Thr Thr Arg Thr Arg Pro
       370                 375                 380
   Thr Asn Arg Thr Ser Ala Thr Thr Arg Thr Arg Ala Thr Arg Val Leu
   385                 390                 395                 400
   Pro Asn Phe Cys Ala Ala Ala Trp Thr Arg Ala Thr Ile Arg Thr Arg
                   405                 410                 415
   Ala Thr Val Arg Thr Arg Thr Pro Thr Arg Thr Arg Ala Ala Ile Arg
                   420                 425                 430
   Thr Arg Ala Thr Arg Leu Arg Gln Pro Ile Pro Cys
           435                 440
<210> 6
   <211> 591
   <212> DNA
   <213> artificial
<220>
   <223> FLAG nucleic acid sequence
<400> 6
   ggacctggtg gatatggacc aggtggtgct ggtcctggtg gttatggtcc tgggggagca      60
   ggacctggtg gttacggccc aggcggcgca ggtccaggcg gatacggtcc aggtggaact     120
   ggaccagggg gctacggccc aggtggaact ggtcctggtg gtgttggacc aggcggagct     180
   ggcccaggcg ggtatggacc tggcggtgca ggaccagggg gggctggtcc tggtggcgct     240
   ggtccaggtg gcgcaggccc tggtggtgct ggtcctggcg gggcaggtcc tgggggctat     300
   gggcctggtg gttccggacc tggtggggct actattattg aggatctcga tatcactatc     360
   gatggtgctg atggaccacc aattactatt tctgaggaac tcactatctc cggtgctggt     420
   ggatctggtc ctggcggcgt gggacctggg ggttcaggtc ctggcggagt tggtccagga     480
   tctttcggac caggtggcgt gggtccaggc ggttctgggc caggtggtgt aggatctggt     540
   ggttctggac aaggtggcgt tagaccatct ggatcaggac caggtggcgt g              591
<210> 7
   <211> 1905
   <212> DNA
   <213> artificial
<220>
   <223> SO1 nucleic acid sequence
<400> 7
   gggcagggag gttatggtgg tctggggggc cagggtgctg gccaaggagg ttatggtggt 60
   ctggggggcc agggtgctgg ccaaggtgca ggagctgctg ctgcagctgc aggtggagcc 120
   gggcagggag gttatggtgg tctggggagt cagggtgctg gtcgtggagg ccaaggtgca 180
   ggagctgcag cagcagctgc aggtggagcc gggcagggag gttatggtgg tctggggagt 240
   cagggcgctg gtcgtggggg actgggtggc caaggtgcag gagcagctgc agctgctgca 300
   ggtggagccg ggcagggagg ttatggtggt ctggggagtc agggtgctgg tcgtggaggc 360
   caaggtgcag gagctgcagc agcagctgca ggtggagccg ggcagggagg ttatggtggt 420
   ctggggagtc agggcgctgg tcgtggggga ctgggtggcc aaggtgcagg agcagctgca 480
   gctgctgcag gtggagccgg gcagggaggt tatggtggtc tggggggcca gggtgctggc 540
   caaggaggtt atggtggtct ggggagtcag ggcgctggtc gtgggggact gggtggccaa 600
   ggtgcaggag ctgctgctgc agctgcaggt ggagccgggc agggaggtct gggagggcag 660
   ggagcgggcc aaggtgcagg agcagctgca gcagctgcag gtggagccgg gcagggaggt 720
   tatggtggtc tggggagtca gggtgctggt cgtggaggcc aaggtgcagg agctgcagca 780
   gcagctgcag gtggagccgg gcagggaggt tatggtggtc tggggggcca gggtgctggc 840
   caaggaggtt atggtggtct ggggagtcag ggcgctggtc gtgggggact gggtggccaa 900
   ggtgcaggag ctgctgctgc agctgcaggt ggagccgggc agggaggtct gggagggcag 960
   ggagcgggcc aaggtgcagg agcagctgca gcagctgcag gtggagccgg gcagggaggt 1020
   tatggtggtc tggggagtca gggcgctggt cgtgggggac tgggtggcca aggtgcagga 1080
   gcagctgcag ctgctgcagg tggagccggg cagggaggtt atggtggtct ggggagtcag 1140
   ggtgctggtc gtggaggcca aggtgcagga gctgcagcag cagctgcagg tggagccggg 1200
   cagggaggtt atggtggtct ggggagtcag ggcgctggtc gtgggggact gggtggccaa 1260
   ggtgcaggag cagctgcagc tgctgcaggt ggagccgggc agggaggtta tggtggtctg 1320
   gggagtcagg gtgctggtcg tggaggccaa ggtgcaggag ctgcagcagc agctgcaggt 1380
   ggagccgggc agggaggtta tggtggtctg gggagtcagg gtgctggtcg tggaggccaa 1440
   ggtgcaggag ctgcagcagc agctgcaggt ggagccgggc agggaggtta tggtggtctg 1500
   gggggccagg gtgctggcca aggaggttat ggtggtctgg ggagtcaggg cgctggtcgt 1560
   gggggactgg gtggccaagg tgcaggagct gctgctgcag ctgcaggtgg agccgggcag 1620
   ggaggtctgg gagggcaggg agcgggccaa ggtgcaggag cagctgcagc agctgcaggt 1680
   ggagccgggc agggaggtta tggtggtctg gggagtcagg gtgctggtcg tggaggccaa 1740
   ggtgcaggag ctgcagcagc agctgcaggt ggagccgggc agggaggtta tggtggtctg 1800
   gggagtcagg gcgctggtcg tgggggactg ggtggccaag gtgcaggagc agctgcagct 1860
   gctgcaggtg gagccggcgg acaagcggcc gcagaacaaa aactc 1905
<210> 8
   <211> 1293
   <212> DNA
   <213> Nephila clavipes
<400> 8
   ggaggacttg gaatccaagg ttccggacga ggaggattag gtggacaagg tgcagttgca      60
   gcagcagcag cagcagctgg aggtgccgta caagtagtac taggtggaca aggtgctgga     120
   caaggagctg gagcagccgc tgcagcagct ggtggtgccg gacaaggagg atatggaggt     180
   cttggaagcc aaggtgctgg acgaggtgga caaggtgcag gcgcacgcac ggcagcagcc     240
   gtaggtgctg gacaaggagg atacggtgga caaggtgccg gacaaggagg ctatggagga     300
   cttggaagcc aaggtgctgg acgaggcgga ttaggtggac aaggtgcagg tgcagcagca     360
   gctgcagcag ctggtagtgc cgaacaagga ttaggtggac aaggtgctgg acaaggagct     420
   ggagcagccg ctgcagcagc tggtggtgcc ggacaaggag gatatggagg tcttggaagc     480
   caaggtgcag gacgaggtgg attaggtgga caaggtgcag gtgcagcagc agcagcagct     540
   ggaggtgctg gacaaggagg atacggtggt cttggtggac aaggtgccgg acaaggaggc     600
   tatggaggac ttggaagcca aggttctggt cgaggaggat taggtggaca gggtgcaggt     660
   gcagcagcag cagcagctgg aggtgctgga caaggaggat taggtggaca aggtgctgga     720
   caaggagctg gagcagccgc tgcagcagct ggtggtgtta gacaaggagg atatggaggt     780
   cttggaagcc aaggtgctgg acgaggtgga caaggtgcag gcgcagccgc agcagcagcc     840
   ggaggtgctg gacaaggagg atatggtggt cttggtggac aaggtgttgg ccgaggtgga     900
   ttaggtggac aaggtgcagg cgcagcggca gctggtggtg ctggacaagg aggatatggt     960
   ggtgttggtt ctggggcgtc tgctgcctct gcagctgcat cccgtttgtc ttctcctcaa    1020
   gctagttcaa gagtttcatc agctgtttcc aacttggttg caagtggtcc tactaattct    1080
   gcggccttgt caagtacaat cagtaacgtg gtttcacaaa taggcgccag caatcctggt    1140
   ctttctggat gtgatgtcct cattcaagct cttctcgagg ttgtttctgc tcttatccac    1200
   atcttaggtt cttccagcat cggccaagtt aactatggtt ccgctggaca agccactcag    1260
   atcgttggtc aatcagttta tcaagcccta ggt                                 1293
<210> 9
   <211> 828
   <212> DNA
   <213> Nephila clavipes
<400> 9
   atgagttggt ccactctagc tttagcgatt ttagcggtgc ttagcaccca atccatctac 60
   gcttctgcac aagcaaggtc tccatggagt gacacagcca cagcagatgc cttcattcag 120
   aatttcttag cagcggtttc gggaagtgga gctttcactt cagatcaact tgatgacatg 180
   tccacgatcg gcgataccat catgtcagca atggacaaaa tggctagaag taacaagagc 240
   tcccaacaca aactgcaagc tctcaacatg gctttcgcct cttccatggc agaaattgca 300
   gcggtggagc aaggtggtat gagcatggca gtaaaaacaa atgcaattgt tgatggcctg 360
   aattcagctt tctacatgac aactggagct gcaaatcctc agtttgtcaa cgaaatgagg 420
   agcttaatta gtatgataag tgcagcttcg gctaatgaag tatcatacgg cggtggagct 480
   tcagcagcag cagcagcagc aggtggttac ggacaaggcc catcaggata tggcccagga 540
   gcatcagctt caagtgccgc agctgcagct gcacctagtg gatacggccc aagccagcaa 600
   ggaccatctg gatccgcagc ggcagcagca gctgcagcac ctagtggata cggtccaaga 660
   cagcaagcac catctggacc cgccgctgca gccgcagccg caggagctgg agggtacgga 720
   ccagcacaaa gaggaccaag tgtatatgga ccatcaggac ctggagcagc agcagcagca 780
   gctgctggcg caggacctgg aggttatgga ccaggacaac aaggacca 828
<210> 10
   <211> 1332
   <212> DNA
   <213> Triticum aestivum
<400> 10
   atggctaagc ggctggtcct ctttgcggca gtagtcatcg ccctcgtggc tctcaccact 60
   gctagccaag tcgcaagaca atatgagcaa actgtggtgc cgcccaaggg cggatccttc 120
   taccctggtg agaccacgcc actgccgcaa ctccaacaag gaatattttg gggaacatct    180
   tcacaaacag tacaagggta ttacccaggc gtaacttctc ctcggcaggg gtcatattat    240
   ccaggccaag cttctccaca acagccagga caagggcaac agcctggcaa atggcaagaa    300
   ccaggacaag ggcaacaatg gtactaccca acttctttgc agcagccagg acaagggcaa    360
   cagtccccag cacacaggac aaaggcaaca accagtacaa gggcaacaac cagaacaagg    420
   gcaacaacca ggacaatggc aacaagggta ctatccaact tctccacaac agctaggaca    480
   agggcaacaa ccaagacaat ggcaacaatc aggacaaggg caacaagggc actacccaac    540
   ttctctacaa cagccaggac aagggcaaca agggcattac ctagcttctc agcagcagcc    600
   aggacaaggg caacaagggc actacccagc ttctcagcag cagccaggac aagggcaaca    660
   agggcactac ccagcttctc agcagcagcc aggacaaggg caacaacagc cagaacaaga    720
   gcaacaacca ggacaagggc aacaaggata ctatccaact tctctggggc agccaggaca    780
   agggcaacag caaggacaag ggcaacaagg gtactaccca acttctctcc agcagccagg    840
   acaagggcaa caagggcact acccagcttc tctgttccag ccaggacaag gacagccagg    900
   acaaaggcaa caaccaggac aagggcaaca tccagaacaa gggaaacaac caggacaagg    960
   gcaacaaggg tactatccaa cttctccaca gcagccagga caagggcaac aactaggaca   1020
   agggcaacaa gggtactacc caacttctcc gcagcagcca ggacaagggc aacaaccagg   1080
   acaagggcaa caagggcact gcccaacgtc cccgcagcag tcaggacaag cgcaacaacc   1140
   aggacaaggc caacaaatag gacaagtgca acaaccagga caagggcaac aagggtacta   1200
   cccaacttct gtgcagcagc ctggacaagg gcaacaatca ggacaagggc aacagtcagg   1260
   acaaggacac caaccaggac aagggcagca atcaggacaa gagcaacaag gctacgacag   1320
   cccataccat gt                                                        1332
<210> 11
   <211> 412
   <212> PRT
   <213> avian influenza
<400> 11
   Ile His Thr Gly Asn Gln His Gln Ala Glu Pro Ile Ser Asn Thr Asn
   1               5                   10                  15
   Phe Leu Thr Glu Lys Ala Val Ala Ser Val Lys Leu Ala Gly Asn Ser
               20                  25                  30
   Ser Leu Cys Pro Ile Asn Gly Trp Ala Val Tyr Ser Lys Asp Asn Ser
           35                  40                  45
   Ile Arg Ile Gly Ser Lys Gly Asp Val Phe Val Ile Arg Glu Pro Phe
       50                  55                  60
   Ile Ser Cys Ser His Leu Glu Cys Arg Thr Phe Phe Leu Thr Gln Gly
   65                  70                  75                  80
   Ala Leu Leu Asn Asp Lys His Ser Asn Gly Thr Val Lys Asp Arg Ser
   85 90 95
   Pro His Arg Thr Leu Met Ser Cys Pro Val Gly Val Ala Pro Ser Pro
   100 105 110
   Tyr Asn Ser Arg Phe Glu Ser Val Ala Trp Ser Ala Ser Ala Cys His
   115 120 125
   Asp Gly Thr Ser Trp Leu Thr Ile Gly Ile Ser Gly Pro Asp Asn Gly
   130 135 140
   Ala Val Ala Val Leu Lys Tyr Asn Gly Ile Ile Thr Asp Thr Ile Lys
   145 150 155 160
   Ser Trp Arg Asn Asn Ile Leu Arg Thr Gln Glu Ser Glu Cys Ala Cys
   165 170 175
   Val Asn Gly Ser Cys Phe Thr Val Met Thr Asp Gly Pro Ser Asn Gly
   180 185 190
   Gln Ala Ser His Lys Ile Phe Lys Met Glu Lys Gly Lys Val Val Lys
   195 200 205
   Ser Val Glu Leu Asp Ala Pro Asn Tyr His Tyr Glu Glu Cys Ser Cys
   210 215 220
   Tyr Pro Asp Ala Gly Glu Ile Thr Cys Val Cys Arg Asp Asn Trp His
   225 230 235 240
   Gly Ser Asn Arg Pro Trp Val Ser Phe Asn Gln Asn Leu Glu Tyr Gln
   245 250 255
   Ile Gly Tyr Ile Cys Ser Gly Val Phe Gly Asp Asn Pro Arg Pro Asn
   260 265 270
   Asp Gly Thr Gly Ser Cys Gly Pro Val Ser Ser Asn Gly Ala Tyr Gly
   275 280 285
   Val Lys Gly Phe Ser Phe Arg Tyr Gly Asn Gly Val Trp Ile Gly Arg
   290 295 300
   Thr Lys Ser Thr Asn Ser Arg Ser Gly Phe Glu Met Ile Trp Asp Pro
   305 310 315 320
   Asn Gly Trp Thr Glu Thr Asp Ser Ser Phe Ser Val Lys Gln Asp Ile
   325 330 335
   Val Ala Ile Thr Asp Trp Ser Gly Tyr Ser Gly Ser Phe Val Gln His
   340 345 350
   Pro Glu Leu Thr Gly Leu Asp Cys Ile Arg Pro Cys Phe Trp Val Glu
           355                 360                 365
   Leu Ile Arg Gly Arg Pro Lys Glu Ser Thr Ile Trp Thr Ser Gly Ser
       370                 375                 380
   Ser Ile Ser Phe Cys Gly Val Asn Ser Asp Thr Val Gly Trp Ser Trp
   385                 390                 395                 400
   Pro Asp Gly Ala Glu Leu Pro Phe Thr Ile Asp Lys
                   405                 410
<210> 12
   <211> 1236
   <212> DNA
   <213> Avian influenza
<400> 12
   attcacacag ggaatcaaca ccaagctgaa ccaatcagca ataccaattt tcttactgag      60
   aaagctgtgg cttcagtaaa attagcgggc aattcatctc tttgccccat taacggatgg     120
   gctgtataca gtaaggacaa cagtataagg atcggctcca agggggatgt gtttgttata     180
   agagagccgt tcatctcatg ctcccacttg gaatgcagaa ctttcttttt gactcaggga     240
   gccttgctga atgacaagca ctccaatggg actgtcaaag acagaagccc tcacagaaca     300
   ttaatgagtt gtcctgtggg tgtggctccc tccccatata actcaaggtt tgagtctgtt     360
   gcttggtcag caagtgcttg ccatgatggc accagttggt tgacaattgg aatttctggc     420
   ccagacaatg gggctgtggc tgtattgaaa tacaatggca taataacaga cactatcaag     480
   agttggagga acaacatatt gagaactcaa gagtctgaat gtgcatgtgt aaatggctct     540
   tgctttactg taatgactga cggaccaagt aatggtcaag catcacataa gatcttcaaa     600
   atggaaaaag ggaaagtggt taaatcagtc gaattggatg ctcctaatta tcactatgag     660
   gaatgctcct gttatcctga tgccggcgaa atcacgtgtg tgtgcaggga taattggcat     720
   ggctcaaatc ggccatgggt atctttcaat caaaatttgg agtatcaaat aggatatata     780
   tgcagtggag ttttcggaga caatccacgc cccaatgatg gaacaggtag ttgcggtccg     840
   gtgtcctcta acggggcata tggggtaaaa ggattttcat ttagatacgg caatggtgtc     900
   tggatcggga gaaccaaaag cactaattcc aggagcggct ttgaaatgat ttgggatcca     960
   aatgggtgga ctgaaacgga cagtagcttt tcagtgaaac aagatatcgt agcaataact    1020
   gattggtcag gatatagcgg gagttttgtc cagcatccag aactgacagg actagattgc    1080
   ataagacctt gtttctgggt tgagttgatc agagggcggc ccaaagagag cacaatttgg    1140
   actagtggga gcagcatatc tttttgtggt gtaaatagtg acactgtggg ttggtcttgg    1200
   ccagacggtg ctgagttgcc attcaccatt gacaag                              1236
<210> 13
   <211> 23
   <212> PRT
   <213> Vicia faba
<210> 14
   <211> 69
   <212> DNA
   <213> Vicia faba
<400> 14
   atggcttcta agccattctt gtctttgttg tccttgtcct tgctcctctt cacttctact      60
   tgtcttgct                                                              69
<210> 15
   <211> 48
   <212> PRT
   <213> Synechocystis spec.
<400> 15
   Ser Pro Glu Ile Glu Lys Leu Ser Gln Ser Asp Ile Tyr Trp Asp Ser
   1               5                   10                  15
   Ile Val Ser Ile Thr Glu Thr Gly Val Glu Glu Val Phe Asp Leu Thr
               20                  25                  30
   Val Pro Gly Pro His Asn Phe Val Ala Asn Asp Ile Ile Val His Asn
           35                  40                  45
<210> 16
   <211> 144
   <212> DNA
   <213> Synechocystis spec.
<400> 16
   tctccagaga ttgagaagct ttctcagtcc gatatctact gggattccat tgtgtccatc      60
   actgaaactg gtgttgaaga ggtgttcgat cttactgttc caggaccaca taacttcgtg     120
   gctaacgata ttatcgtgca caac                                            144
<210> 17
   <211> 5
   <212> PRT
   <213> Synechocystis spec.
<400> 17
   Ser Ile Glu Gln Asp
   1 5
<210> 18
   <211> 15
   <212> DNA
   <213> Symechocystis spec.
<400> 18
   tccattgagc aagat 15
<210> 19
   <211> 15
   <212> PRT
   <213> artificial
<220>
   <223> spacer sequence
<400> 19
   Gly Gly Gly Gly Ser Gly Gly Gly Gly Ser Gly Gly Gly Gly Ser
   1 5 10 15
<210> 20
   <211> 45
   <212> DNA
   <213> artificial
<220>
   <223> spacer nucleic acid sequence
<400> 20
   ggtggtggtg gttcaggtgg tggtggaagt ggtggtggcg gatct 45
<210> 21
   <211> 11
   <212> PRT
   <213> artificial
<220>
   <223> myc-tag amino acid sequence
<400> 21
   Glu Gln Lys Leu Ile Ser Glu Glu Asp Leu Asn
   1 5 10
<210> 22
   <211> 33
   <212> DNA
   <213> artificial
<220>
   <223> c-myc tag nucleic acid sequence
<400> 22
   gaacaaaagc ttatttctga agaggatctc aac 33
<210> 23
   <211> 4
   <212> PRT
   <213> Synechocystis spec.
<400> 23
   Arg Glu Ser Gly
   1
<210> 24
   <211> 12
   <212> DNA
   <213> Synechocystis spec.
<400> 24
   agagagtccg gt 12
<210> 25
   <211> 106
   <212> PRT
   <213> Synechocystis spec.
<400> 25
   Cys Ile Ser Gly Asp Ser Leu Ile Ser Leu Ala Ser Thr Gly Lys Arg
   1               5                   10                  15
   Val Ser Ile Lys Asp Leu Leu Asp Glu Lys Asp Phe Glu Ile Trp Ala
               20                  25                  30
   Ile Asn Glu Gln Thr Met Lys Leu Glu Ser Ala Lys Val Ser Arg Val
           35                  40                  45
   Phe Cys Thr Gly Lys Lys Leu Val Tyr Ile Leu Lys Thr Arg Leu Gly
       50                  55                  60
   Arg Thr Ile Lys Ala Thr Ala Asn His Arg Phe Leu Thr Ile Asp Gly
   65                  70                  75                  80
   Trp Lys Arg Leu Asp Glu Leu Ser Leu Lys Glu His Ile Ala Leu Pro
                   85                  90                  95
   Arg Lys Leu Glu Ser Ser Ser Leu Gln Leu
               100                 105
<210> 26
   <211> 318
   <212> DNA
   <213> Synechocystis spec.
<400> 26
   tgtatttctg gtgattctct tatttccctc gcttccactg gaaagagggt ttccattaag 60
   gatcttctcg atgagaagga tttcgagatc tgggctatta acgagcagac tatgaagctt 120
   gagtctgcta aggtttccag ggttttctgt actggaaaga agctcgttta catcctcaag 180
   actaggctcg gaaggactat taaggctact gctaaccacc gtttcctcac tattgatgga 240
   tggaagaggc ttgatgagtt gtctctcaaa gagcacattg ctttgcctag gaagttggag 300
   tcatcttctc ttcaatta 318
<210> 27
   <211> 4
   <212> PRT
   <213> artificial
<220>
   <223> ER retention signal
<400> 27
   Lys Asp Glu Leu
   1
<210> 28
   <211> 12
   <212> DNA
   <213> artificial
<220>
   <223> ER retention signal nucleic acid sequence
<400> 28
   aaagacgaac tc 12
<210> 29
   <211> 39
   <212> DNA
   <213> artificial
<220>
   <223> forward primer
<400> 29
   agcgtctcgg atccattcac acagggaatc aacaccaag 39
<210> 30
   <211> 34
   <212> DNA
   <213> artificial
<220>
   <223> reverse primer
<400> 30
   agcgtctcgg atcccttgtc aatggtgaat ggca 34
<210> 31
   <211> 5
   <212> PRT
   <213> artificial
<220>
   <223> amino acid sequence of the ELP repeat
<220>
   <221> VARIANT
   <222> (4)..(4)
   <223> X is any amino acid except proline
<400> 31
   Val Pro Gly Xaa Gly
   1               5
<210> 32
   <211> 1532
   <212> DNA
   <213> artificial
<220>
   <223> nucleic acid sequence coding for 100 repeats of the ELP pentamer
<400> 32
   ctcgagatgg gccacggcgt gggtgttccg ggcgtgggtg ttccgggtgg cggtgtgccg      60
   ggcgcaggtg ttcctggtgt aggtgtgccg ggtgttggtg tgccgggtgt tggtgtacca     120
   ggtggcggtg ttccgggtgc aggcgttccg ggtggcggtg tgccgggcgt gggtgttccg     180
   ggcgtgggtg ttccgggtgg cggtgtgccg ggcgcaggtg ttcctggtgt aggtgtgccg     240
   ggtgttggtg tgccgggtgt tggtgtacca ggtggcggtg ttccgggtgc aggcgttccg     300
   ggtggcggtg tgccgggcgt gggtgttccg ggcgtgggtg ttccgggtgg cggtgtgccg     360
   ggcgcaggtg ttcctggtgt aggtgtgccg ggtgttggtg tgccgggtgt tggtgtacca     420
   ggtggcggtg ttccgggtgc aggcgttccg ggtggcggtg tgccgggcgt gggtgttccg     480
   ggcgtgggtg ttccgggtgg cggtgtgccg ggcgcaggtg ttcctggtgt aggtgtgccg     540
   ggtgttggtg tgccgggtgt tggtgtacca ggtggcggtg ttccgggtgc aggcgttccg     600
   ggtggcggtg tgccgggcgt gggtgttccg ggcgtgggtg ttccgggtgg cggtgtgccg     660
   ggcgcaggtg ttcctggtgt aggtgtgccg ggtgttggtg tgccgggtgt tggtgtacca     720
   ggtggcggtg ttccgggtgc aggcgttccg ggtggcggtg tgccgggcgt gggtgttccg     780
   ggcgtgggtg ttccgggtgg cggtgtgccg ggcgcaggtg ttcctggtgt aggtgtgccg     840
   ggtgttggtg tgccgggtgt tggtgtacca ggtggcggtg ttccgggtgc aggcgttccg     900
   ggtggcggtg tgccgggcgt gggtgttccg ggcgtgggtg ttccgggtgg cggtgtgccg     960
   ggcgcaggtg ttcctggtgt aggtgtgccg ggtgttggtg tgccgggtgt tggtgtacca    1020
   ggtggcggtg ttccgggtgc aggcgttccg ggtggcggtg tgccgggcgt gggtgttccg    1080
   ggcgtgggtg ttccgggtgg cggtgtgccg ggcgcaggtg ttcctggtgt aggtgtgccg    1140
   ggtgttggtg tgccgggtgt tggtgtacca ggtggcggtg ttccgggtgc aggcgttccg    1200
   ggtggcggtg tgccgggcgt gggtgttccg ggcgtgggtg ttccgggtgg cggtgtgccg    1260
   ggcgcaggtg ttcctggtgt aggtgtgccg ggtgttggtg tgccgggtgt tggtgtacca    1320
   ggtggcggtg ttccgggtgc aggcgttccg ggtggcggtg tgccgggcgt gggtgttccg    1380
   ggcgtgggtg ttccgggtgg cggtgtgccg ggcgcaggtg ttcctggtgt aggtgtgccg    1440
   ggtgttggtg tgccgggtgt tggtgtacca ggtggcggtg ttccgggtgc aggcgttccg    1500
   ggtggcggtg tgccgggcgg gctggcggcc gc                                  1532